# EUROPEAN PATENT SPECIFICATION

(11) **EP 0 760 362 B1**
(45) Date of publication and mention of the grant of the patent: **14.08.2002**
(21) Application number: 95918729.5
(22) Date of filing: 17.05.1995
(51) Int. Cl.: C07C 211/24, C07C 211/27, C07C 211/29, C07C 211/53, C07C 255/30, C07C 275/06, C07C 275/26, C07C 275/28

(54) **DIAMINOMETHYLIDENE DERIVATIVE**
DIAMINOMETHYLIDEN-DERIVATE
DERIVES DE DIAMINOMETHYLIDENE

(30) Priority: 18.05.1994 JP 10357094
(43) Date of publication of application: 05.03.1997
(73) Proprietor: NISSHIN SEIFUN GROUP INC., Chiyoda-ku, Tokyo (JP)
(72) Inventor: KIKUCHI, Haruhiko Nisshin Flour Milling Co., Ltd., home Ohimachi, Iruma-gun Saitama 356 (JP); SATOH, Hiroaki Nisshin Flour Milling Co., Ltd., 5-chome Ohimachi, Iruma-gun Saitama 356 (JP); FUKUTOMI, Ryuta Nisshin Flour Milling Co., Ltd., 5-chome Ohimachi, Iruma-gun Saitama 356 (JP); INOMATA, Kohei Nisshin Flour Milling Co., Ltd., 5-chome Ohimachi, Iruma-gun Saitama 356 (JP); SUZUKI, Masashi Nisshin Flour Milling Co., Ltd., 5-chome Ohimachi, Iruma-gun Saitama 356 (JP); HAGIHARA, Koichiro Nisshin Flour Milling Co.,Ltd., chome Ohimachi, Iruma-gun Saitama 356 (JP); ARAI, Takeo Nisshin Flour Milling Co., Ltd., 5-chome Ohimachi, Iruma-gun Saitama 356 (JP); MINO, Setsuko Nisshin Flour Milling Co., Ltd., 5-chome Ohimachi, Iruma-gun Saitama 356 (JP); EGUCHI, Haruko Nisshin Flour Milling Co., Ltd., 5-chome Ohimachi, Iruma-gun Saitama 356 (JP)
(74) Representative: Marshall, Monica Anne
(86) International application number: JP9500938
(87) International publication number: WO9531431

(56) References cited:
- JP-A- 2 202 890
- JP-A- 4 077 464
- JP-A- 5 194 359
- JP-A- 50 117 790
- JP-A- 60 081 177
- JP-A- 63 301 879
- US-A- 3 585 142
- US-A- 4 071 524
- US-A- 4 416 669

## Description

This invention relates to novel diaminomethylidene derivatives and pharmacologically acceptable acid addition salts and quaternary ammonium salts thereof.

More specifically, this invention relates to new diaminomethylidene derivatives having a promoting action of the release of acetylcholine in digestive tracts, thus being useful for the treatment of digestive tract disorders derived from chronic gastritis, diabetes mellitus, post-gastrectomy and peptic ulcer and digestive tract diseases including reflux esophagitis, irritable bowel syndrome and spurious ileus, as well as a gastrointestinal prokinetic agent which comprises as an active ingredient the said derivatives.

### Background Art

The abnormality in function of a gastrointestinal mobility by various causes such as chronic gastritis, diabetes, post-gastrectomy, peptic ulcer and others could result in the reflux of the gastric content into the esophagus, delayed emptying of the gastric content and the depressed function of the small and large intestines.

This can lead to appearances of nausea, vomiting, heartburn, anorexia, abdominal distention, epigastric dysphoria, abdominaglia, constipation and further reflux espophagitis. One cause of the diseases such as irritable bowel syndrome and spurious ileus has been considered to be the depression in gastrointestinal motility.

The agents for the treatment of these conditions and diseases include direct cholinergic agent (e.g. Aclatonium Napadisilate) or Dopamine antagonist (e.g. Doperidone). However, it is well-known that these known agents have problems in their effects and side-effects, which may include, for example, diarrhea and extrapyramidal syndrome.

It is well-known that acetylcholine is the neurotransmitter participating in the control of gastrointestinal motility. Accordingly, it may be considered that a compound capable of promoting the release of acetylcholine in the digestive tract may be a gastrointestinal prokinetic agent showing far more effectiveness and less side effects. In view of this, it has been desired to find out such a type of compounds and develop a process for obtaining them.

EP-A-0377967 describes compounds of formula: 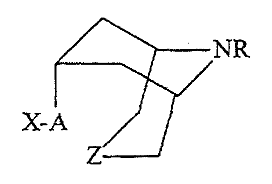 (wherein X is a phenyl group or a monocyclic 5 or 6 membered heteroaryl group, either of which groups is optionally fused to a saturated or unsaturated 5-7 membered carbocyclic or heterocyclic ring; A is a linking moiety; Z is oxygen or sulphur; and R is methyl or ethyl) and their pharmaceutically acceptable salts. The compounds are described as having 5-HT₃ receptor antagonist activity.

EP-A-0526313 describes urea derivatives of the general formula: 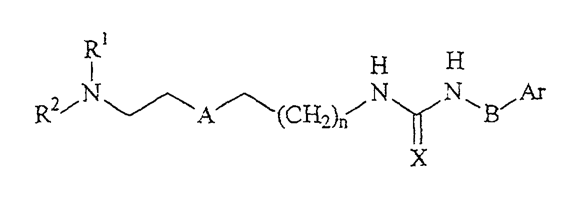 in which R₁ represents a C₁-C₄ alkyl group; R₂ represents a C₅-C₇ cycoalkyl group, a cycoalkyl methyl group wherein the cycoalkyl radical contains 5 to 7 carbon atoms, a benzyl group or a benzyl group in which the aromatic ring carries a C₁-C₄ alkyl group, a C₁-C₄ alkoxy group, a halogen atom or a nitro group; A represents an oxygen atom or a methylene radical; n represents I or 2; X represents an oxygen or sulphur atom, and B represents a direct bond, a methylene radical or a carbonyl radical, as well as the therapeutically acceptable salts of these molecules. The urea derivatives are said to inhibit acetylcholinesterase and are said to be useful for the treatment of illnesses such as myasthenia, memory dysfunction and dementia, such as senile dementia and Alzheimer's disease.

EP-A-0092647 describes compounds of formula: 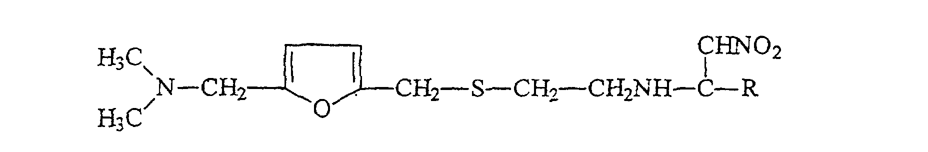 (in which R is a piperidine group, 4-methyl piperidine, (3-N-ethyl-piperidinyl) amine, 4-(2-hydroxyethyl)-1-piperazine, 4-benzyl piperidine, 4-benzyl piperazine, ethylene amine, cyclopropylene amine, cyclohexyl amine, 1,4-cyclohexadienyl amine, 1,4-cyclohexadienyl-2-methyl amine, 1,4-cyclohexadienyl-2-ethyl amine, hexamethylene amine, (N-hexamethylene amine) amine, cycloheptyl amine, cyclopentyl amine, pyrrolidine, morpholine, endo-2-norbornyl amine, norbornen-2-yl amine) and their pharmaceutically acceptable salts and their use in the treatment of ulcers and skin allergy symptons.

US-A-4071524 describes compounds of formula: 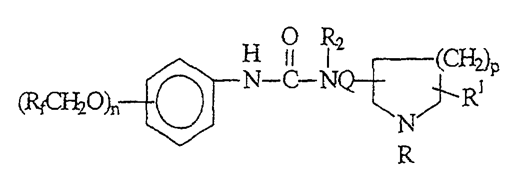 wherein R_{f} is a perfluoroalkyl radical containing one to three carbon atoms, n is one to three, *p* is one or two, Q is a carbon-nitrogen bond, methylene or methylmethylene, R and R¹ are hydrogen, methyl or ethyl, and R² is lower alkyl or hydrogen, provided that when Q is a carbon-nitrogen bond it is bonded to the 3 position of the heterocyclic ring and when it is methylene or methylmethylene it is bonded to the 2 position, and pharmaceutically acceptable salts thereof. These compounds are suitable as active antiarrhythmic agents.

### Disclosure of Invention

According to one aspect of the invention, there is provided a diaminomethylidene derivative represented by formula (1) 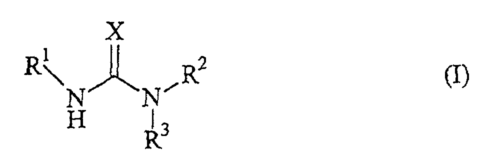 wherein
R¹ is a hydrogen atom, a C₁-C₆ alkyl group, a C₃-C₆ cycloalkyl group, a C₃-C₆ cycloalkyl C₁-C₄ alkyl group, an aryl group or an aryl C₁-C₄ alkyl group, in which the aryl moiety of the aryl group or aryl C₁-C₄ alkyl group may be optionally substituted with a halogen atom, a C₁-C₆ alkyl group, a halo C₁-C₆ alkyl group, a C₁-C₆ alkoxy group, a C₁-C₆ alkoxycarbonyl group, a phenyl group or an amino group;
X is O, S, CHNO₂, C(COOR⁴)₂, C(COOR⁴) CN or C(CN)₂, in which R⁴ is a C₁-C₆ alkyl group, a C₃-C₆ cycloalkyl group, an aryl group, or an aryl C₁-C₄ alkyl group; and
R² is a group of the following general formula (II) or (III) 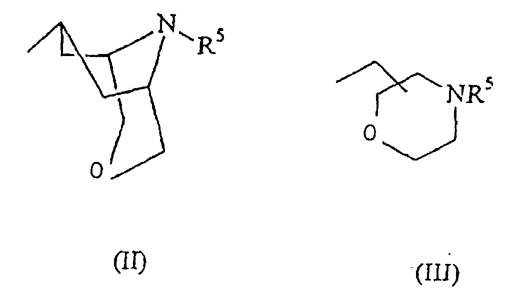
X is CHNO₂, C(COOR⁴)₂, C(COOR⁴) CN or C(CN)₂, in which R⁴ is as defined above; and
R² is a group of the following general formula (IV) 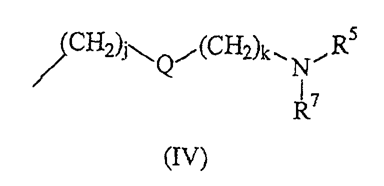 (wherein j is 0-3, k is 0-3, and the sum of j and k is 1-6, and Q is O);
R³ is a hydrogen atom, a C₁-C₆ alkyl group or a C₁-C₆ alkoxy C₂-C₆ alkyl group;
R⁵ is an aryl C₁-C₄ alkyl group, a heteroaryl C₁-C₄ alkyl group, an aryloxy C₂-C₆ alkyl group, or a pyrrolidinylcarbonyl C₁-C₄ alkyl group, in which the aryl moiety of the said groups may be optionally substituted with a halogen atom, a C₁-C₆ alkyl group, a halo C₁-C₆ alkyl group, a C₁-C₆ alkoxy group, a C₁-C₆ alkoxycarbonyl group, a phenyl group or an amino group; and
R⁷ is a hydrogen atom, a C₁-C₆ alkyl group or a C₁-C₄ alkoxy C₂-C₄ alkyl group; or a pharmacologically acceptable salt thereof.

These diaminomethylidene derivatives can promote the release of aceytlcholine.

In formula (I) for the diaminomethylidene derivatives of this invention, the C₁-C₆ alkyl group represented by R¹ may include methyl, ethyl, n-propyl, isopropyl, n-butyl, isobutyl, n-pentyl, neo-pentyl, n-hexyl and the like; the C₃-C₆ cycloalkyl group may include cyclopropyl, cyclopentyl or cyclohexyl; the aryl group may include phenyl, naphthyl, o-fluorophenyl, m-fluorophenyl, p-fluorophenyl, o-chlorophenyl, m-chlorophenyl, p-chlorophenyl, 3,4-dichlorophenyl, o-methylphenyl, m-methylphenyl, p-methylphenyl, o-ethylphenyl, m-ethylphenyl, p-ethylphenyl, o-methoxyphenyl, m-methoxyphenyl, p-methoxyphenyl, o-ethoxyphenyl, m-ethoxyphenyl, p-ethoxyphenyl, 3,4-dimethoxyphenyl, o-methoxycarbonylphenyl, m-methoxycarbonylphenyl, p-methoxycarbonylphenyl, o-aminophenyl, m-aminophenyl, p-aminophenyl and the like; the C₃-C₆ cycloalkyl C₁-C₄ alkyl group may include cyclopropylmethyl, cyclohexylmethyl and the like; the aryl C₁-C₄ alkyl group may include benzyl, phenethyl, o-fluorobenzyl, m-fluorobenzyl, p-fluorobenzyl, o-chlorobenzyl, m-chlorobenzyl, p-chlorobenzyl, 3,4-dichlorobenzyl, o-methylbenzyl, m-methylbenzyl, p-methylbenzyl, o-trifluoromethylbenzyl, m-trifluoromethylbenzyl, p-trifluoromethylbenzyl, o-methoxybenzyl, m-methoxybenzyl, p-methoxybenzyl, 3,4-dimethoxybenzyl, o-methoxycarbonylbenzyl, m-methoxycarbonylbenzyl, p-methoxycarbonylbenzyl, biphenyl-2-ylmethyl, biphenyl-3-ylmethyl, biphenyl-4-ylmethyl, o-aminobenzyl, m-aminobenzyl, p-aminobenzyl, o-fluorophenethyl, m-fluorophenethyl, p-fluorophenethyl, o-chlorophenethyl, m-chlorophenethyl, p-chlorophenethyl, 3,4-dichlorophenethyl, o-methylphenethyl, m-methylphenethyl, p-methylphenethyl, o-trifluoromethylphenethyl, m-trifluoromethylphenethyl, p-trifluoromethylphenethyl, o-methoxyphenethyl, m-methoxyphenethyl, p-methoxyphenethyl, 3,4-dimethoxyphenethyl, o-methoxycarbonylphenethyl, m-methoxycarbonylphenethyl, p-methoxycarbonylphenethyl, 2-(biphenyl-2-yl)ethyl, 2-(biphenyl-3-yl)ethyl, 2-(biphenyl-4-yl)ethyl, o-aminophenethyl, m-aminophenethyl, p-aminophenethyl and the like; the C₁-C₆ alkyl group represented by R⁴ may include methyl, ethyl, n-propyl, isopropyl, n-butyl, isobutyl, n-pentyl, neo-pentyl, n-hexyl and the like; the C₃-C₆ cycloalkyl group may include cyclopropyl, cyclopentyl, cyclohexyl and the like; the aryl group may include phenyl, naphthyl and the like; the aryl C₁-C₄ alkyl group may include benzyl, phenethyl and the like; the C₁-C₆ alkyl group represented by R³ may include methyl, ethyl, n-propyl, isopropyl, n-butyl, isobutyl, n-pentyl, neo-pentyl, n-hexyl and the like; the C₁-C₆ alkoxy C₂-C₆ alkyl group may include 2-methoxyethyl, 2-ethoxyethyl and the like.

The aryl C₁-C₄ alkyl group represented by R⁵ in formulae (II) - (IV) may include benzyl, phenethyl, o-fluorobenzyl, m-fluorobenzyl, p-fluorobenzyl, o-chlorobenzyl, m-chlorobenzyl, p-chlorobenzyl, 3,4-dichlorobenzyl, o-methylbenzyl, m-methylbenzyl, p-methylbenzyl, o-trifluoromethylbenzyl, m-trifluoromethylbenzyl, p-trifluoromethylbenzyl, o-methoxybenzyl, m-methoxybenzyl, p-methoxybenzyl, 3,4-dimethoxybenzyl, o-methoxycarbonylbenzyl, m-methoxycarbonylbenzyl, p-methoxycarbonylbenzyl, biphenyl-2-ylmethyl, biphenyl-3-ylmethyl, biphenyl-4-ylmethyl, o-aminobenzyl, m-aminobenzyl, p-aminobenzyl, o-fluorophenethyl, m-fluorophenethyl, p-fluorophenethyl, o-chlorophenethyl, m-chlorophenethyl, p-chlorophenethyl, 3,4-dichlorophenethyl, o-methylphenethyl, m-methylphenethyl, p-methylphenethyl, o-trifluoromethylphenethyl, m-trifluoromethylphenethyl, p-trifluoromethylphenethyl, o-methoxyphenethyl, m-methoxyphenethyl, p-methoxyphenethyl, 3,4-dimethoxyphenethyl, o-methoxycarbonylphenethyl, m-methoxycarbonylphenethyl, p-methoxycarbonylphenethyl, 2-(biphenyl-2-yl)ethyl, 2-(biphenyl-3-yl)ethyl, 2-(biphenyl-4-yl)ethyl, o-aminophenethyl, m-aminophenethyl, p-aminophenethyl and the like; the heteroaryl C₁-C₄ alkyl group may include 2-pyridylmethyl, 3-pyridylmethyl, 4-pyridylmethyl, lH-indol-3-ylethyl and the like; the aryloxy C₂-C₆ alkyl group may include phenoxy-2-ethyl, phenoxy-3-propyl, 2-fluorophenoxy-3-propyl, 3-fluorophenoxy-3-propyl, 4-fluorophenoxy-3-propyl, 2-chlorophenoxy-3-propyl, 3-chlorophenoxy-3-propyl, 4-chlorophenoxy-3-propyl, 3,4-dichlorophenoxy-3-propyl and the like; the pyrrolidinylcarbonyl C₁-C₄ alkyl group may include pyrrolidinylcarbonylmethyl group and the like.

The C₁-C₆ alkyl group represented by R⁷ may include methyl, ethyl, n-propyl, isopropyl, n-butyl, isobutyl, n-pentyl, neo-pentyl, n-hexyl and the like; the C₁-C₄ alkoxy C₂-C₄ alkyl group may include methoxyethyl, ethoxyethyl and the like.

The compounds represented by formula (I) of this invention may be prepared according to the process as explained below.

That is to say, the compound represented by formula (I) may be prepared by reacting a methylthioaminomethylidene compound represented by formula (XI) 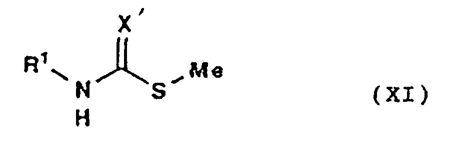 wherein R¹ has the same meaning as above and X' is CHNO₂, C(COOR⁴)₂, C(COOR⁴)CN or C(CN)₂, and R⁴ is a C₁-C₆ alkyl group, a C₃-C₆ cycloalkyl group, an aryl group or an aryl C₁-C₄ alkyl group, with an amino compound represented by formula (XII)

R²R³NH (XII)

wherein R² and R³ have the same meanings as above.

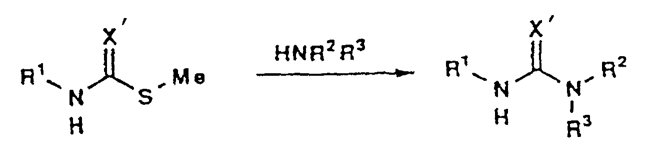

Examples of the methylthioaminomethylidene compounds may illustratively include 2-benzylamino-2-methylthio-1,1-ethylenedicarbonitrile, 2-methylamino-2-methylthio-1,1-ethylenedicarbonitrile, 2-phenylamino-2-methylthio-1,1-ethylenedicarbonitrile, 2-cyclohexylamino-2-methylthio-1,1-ethylenedicarbonitrile, 2-isobutylamino-2-methylthio-1,1-ethylenedicarbonitrile, ethyl 3-cyclohexylamino-3-methylthio-2-cyanoacrylate, ethyl 2-cyano-3-methylamino-3-methylthioacrylate, ethyl 2-cyano-3-phenylamino-3-methylthioacrylate, ethyl 2-cyano-3-isobutylamino-3-methylthioacrylate, ethyl 3-benzylamino-2-cyano-3-methylthioacrylate, ethyl 2-cyano-3-(p-methoxyphenyl)amino-3-methylthioacrylate, N-(1-methylthio-2-nitrovinyl)-N-methylamine, 2-[4-(p-fluorobenzyl)-2-morpholinylmethylamino]-2-methylthio-1,1-ethylenedicarbonitrile and the like.

Examples of the amino compounds may illustratively include 2-aminomethyl-4-(p-fluorobenzyl)morpholine, endo-7-amino-9-(p-fluorobenzyl)-9-aza-3-oxabicyclo[3.3.1]-nonane, endo-7-amino-9-[3-(p-fluorophenoxypropyl)]-9-aza-3-oxabicyclo[3.3.1]nonane and the like.

The reaction of the aminomethylidene compound with the amino compound may be carried out using an amount of the amino compound of 0.2-5 moles, preferably 0.5-2 moles, per mole of the methylthioaminomethylidene compound. The reaction may be preferably carried out in an organic solvent at a temperature ranging from -78°C to a boiling point of the solvent used, and the reaction may proceed very easily so that the reaction may proceed usually at a temperature from room temperature to about 45°C under warming. The organic solvent which may be employed herein may include dimethylformamide, acetonitrile, dimethyl sulfoxide, acetone, ethyl acetate, ether, chloroform, methylene chloride, tetrahydrofuran, dioxane, toluene, benzene and the like and dimethylformamide or acetonitrile may be preferably used.

The desired product thus prepared may be isolated and purified from the reaction mixture by way of well-known purification means such as concentration, extraction, chromatographic purification, recrystallization and others.

Further, the compounds represented by formula (I) wherein X is 0 or S may be prepared by the reaction of an isocyanate or isothiocyanate compound represented by formula (XIII)

R¹NCX" (XIII)

wherein R² has the same meaning as above and X" is O or S, with an amino compound represented by formula (XII)

R²R³NH (XII)

wherein R² represents a group of formula (II) or (III) above and R³ has the same meanings as above.

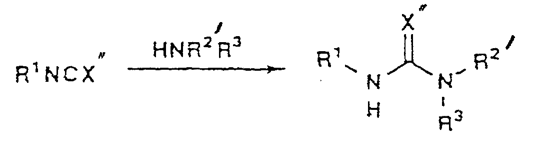

Examples of the isocyanate compounds may illustratively include phenyl isocyanate, methyl isocyanate, benzyl isocyanate, cyclohexyl isocyanate, isobutyl isocyanate, p-methoxyphenyl isocyanate and the like. Examples of the isothiocyanate compounds may illustratively include phenyl isothiocyanate, methyl isothiocyanate, benzyl isothiocyanate, cyclohexyl isothiocyanate, isobutyl isothiocyanate, p-methoxyphenyl isothiocyanate and the like.

Examples of the amino compounds may illustratively include 2-aminomethyl-4-(p-fluorobenzyl)morpholine, endo-7-amino-9-(p-fluorobenzyl)-9-aza-3-oxabicyclo[3.3.1]-nonane, endo-7-amino-9-[3-(p-fluorophenoxypropyl)]-9-aza-3-oxabicyclo[3.3.1]nonane and the like.

The reaction of the isocyanate or isothiocyanate compound with the amino compound may be carried out using an amount of the amino compound of 0.2-5 moles, preferably 0.5-2 moles, per mole of the isocyanate or isothiocyante compound. The reaction may be preferably carried out in an organic solvent at a temperature ranging from room temperature to a boiling point of the solvent used, and the reaction may proceed very easily so that the reaction may be proceed usually at a temperature from room temperature to about 45°C by under slightly warming. The organic solvent which may be employed may include dimethylformamide, acetonitrile, dimethylsulfoxide, acetone, ethyl acetate, ether, chloroform, methylene chloride, tetrahydrofuran, dioxane, toluene, benzene and the like and dimethylformamide or acetonitrile may be preferably used. Illustrative compounds of the present invention may be given below in terms of their chemical names;
2-benzylamino-2-[4-(p-fluorobenzyl)-2-morpholinylmethylamino]-1,1-ethylenedicarbonitrile,
2-cyclohexylamino-2-[4-(p-fluorobenzyl)-2-morpholinylmethylamino]-1,1-ethylenedicarbonitrile,
2-[4-(p-fluorobenzyl)-2-morpholinylmethylamino]-2-phenylamino-1,1-ethylenedicarbonitrile,
2-[4-(p-fluorobenzyl)-2-morpholinylmethylamino]-2-methylamino-1,1-ethylenedicarbonitrile,
2-[4-(p-fluorobenzyl)-2-morpholinylmethylamino]-2-isobutylamino-1,1-ethylenedicarbonitrile,
ethyl 3-benzylamino-3-[4-(p-fluorobenzyl)-2-morpholinylmethylamino]-2-cyanoacrylate,
ethyl 3-[4-(p-fluorobenzyl)-2-morpholinylmethylamino]-3-phenylamino-2-cyanoacrylate,
ethyl 3-[4-(p-fluorobenzyl)-2-morpholinylmethylamino]-3-isobutylamino-2-cyanoacrylate,
ethyl 3-cyclohexylamino-3-[4-(p-fluorobenzyl)-2-morpholinylmethylamino]-2-cyanoacrylate,
ethyl 3-[4-(p-fluorobenzyl)-2-morpholinylmethylamino]-3-(p-methoxybenzyl)amino-2-cyanoacrylate,
ethyl 3-[4-(p-fluorobenzyl)-2-morpholinylmethylamino]-3-methylamino-2-cyanoacrylate,
ethyl 3-cyclohexylamino-3-[7-(p-fluorophenyl)-6-aza-3-oxaheptylamino]-2-cyanoacrylate,
2-(p-fluorobenzylamino)-2-[4-(p-fluorobenzyl)-2-morpholinylmethylamino]-1,1-ethylenedicarbonitrile,
ethyl 2-cyano-3-[endo-9-(p-fluorobenzyl)-9-aza-3-oxabicyclo-[3.3.1]non-7-ylamino]-3-methylaminoacrylate,
2-[endo-9-(p-fluorobenzyl)-9-aza-3-oxabicyclo[3.3.1]non-7-ylamino]-2-methylamino-1,1-ethylenedicarbonitrile,
2-[endo-9-[3-(p-fluorophenoxy)propyl]-9-aza-3-oxabicyclo-[3.3.1]non-7-ylamino]-2-methylamino-1,1-ethylenedicarbonitrile,
N-[endo-9-(p-fluorobenzyl)-9-aza-3-oxabicyclo[3.3.1]non-7-yl]-N'-methyl-2-nitrovinylidene-1,1-diamine,
1-[4-(p-fluorobenzyl)-2-morpholinylmethyl]-3-phenylurea, 1-[4-(p-fluorobenzyl)-2-morpholinylmethyl]-3-methylurea,
1-[endo-9-(p-fluorobenzyl)-9-aza-3-oxabicyclo[3.3.1]non-7-yl]-3-methylurea,
2-[4-(p-chlorobenzyl)-2-morpholinylmethylamino]-2-methylamino-1,1-ethylenedicarbonitrile,
2-[4-(3,4-dichlorobenzyl)-2-morpholinylmethylamino]-2-methylamino-1,1-ethylenedicarbonitrile,
2-[4-(p-trifluoromethylbenzyl)-2-morpholinylmethylamino]-2-methylamino-1,1-ethylenedicarbonitrile,
2-(4-benzyl-2-morpholinylmethylamino)-2-methylamino-1,1-ethylenedicarbonitrile,
2-methylamino-2-[4-(4-pyridylmethyl)-2-morpholinylmethylamino]-1,1-ethylenedicarbonitrile,
2-[4-(biphenyl-4-ylmethyl)-2-morpholinylmethylamino]-2-methylamino-1,1-ethylenedicarbonitrile,
2-[4-(p-methoxybenzyl)-2-morpholinylmethylamino]-2-methylamino-1,1-ethylenedicarbonitrile,
2-[4-(p-fluorobenzyl)-3-morpholinylmethylamino]-2-methylamino-1,1-ethylenedicarbonitrile,
2-[endo-9-(p-fluorobenzyl)-9-aza-3-oxabicyclo[3.3.1]non-7-ylamino]-2-methylamino-1,1-ethylenedicarbonitrile,
2-[exo-9-(p-fluorobenzyl)-9-aza-3-oxabicyclo[3.3.1]non-7-ylamino]-2-methylamino-1,1-ethylenedicarbonitrile,

The compounds of the present invention as described above have a remarkable gastrointestinal prokinetic action, thus being useful as a therapeutic agent for digestive tract diseases, as will be proved in the following Examples.

The present compounds represented by formula (I) may be converted, if desired, to the corresponding acid addition salts with pharmaceutically acceptable acids. The acid addition salts are included within the scope of this invention, which include, for example, the salts with inorganic acids such as hydrochloric acid, hydrobromic acids, sulfuric acid, nitric acid, phosphoric acid and the like, or the salts with organic acids such as acetic acid, succinic acid, oxalic acid, malic acid, tartaric acid and the like.

The compounds represented by formula (I) when they are to be applied as medicines may be formulated to various dosage forms. More specifically, the preparations thus formed may be administered orally in a dosage form of tablets, sugar-coated tablets, hard capsules, soft capsules or in the form of solutions, emulsions, suspensions and the like. In parenteral administration, they may be given in the form of injections.

In preparing such pharmaceutical preparations, the present compounds may be formulated using those additives conventionally used for formulation, such as excipients, stabilizers, preservatives, solubilizers, wetting agents, emulsions, lubricants, sweeteners, colorants, flavorings, tonicity agents, buffers, antioxidants and the like.

Route and dosage of administration for the present gastrointestinal prokinetic agent are not specifically limited and are appropriately chosen depending upon various dosage forms, sex of the patients, severity of the diseases. Daily dose of the active ingredient is 0.001 mg to 1000 mg.

According to a further aspect of the present invention there is a provided a gastrointestinal prokinetic agent which comprises as active ingredient a compound of formula (I) or a pharmacologically acceptable salt thereof, if necessary, in admixture with a pharmaceutically acceptable additive.

According to another aspect the invention provides a compound of the formula (I) or a pharmaceutically acceptable salt thereof for use in the therapy of digestive tract disorders derived from chronic gastritis, diabetes mellitus, post-gastrectomy and peptic ulcer and digestive tract diseases including reflux esophagitis, irritable bowel syndrome and spurious ileus.

A further aspect of the present invention provides use of the compound of formula (I) or a pharmacologically acceptable salt thereof in the manufacture of a medicament for use in the treatment of digestive tract disorders derived from chronic gastritis, diabetes mellitus, post-gastrectomy and peptic ulcer and digestive tract diseases including reflux esophagitis, irritable bowel syndrome and spurious ileus.

This invention will be explained in greater detail by way of the following Preparation Examples and Examples. However, it is to be noted that Preparation Examples are given to explain the synthetic examples in regard to the starting compounds to be used for preparing the present compounds, while the Examples are given for explaining the synthesis and use of this invention as medicines. Those Preparation Examples and Examples are given simply for the purpose of explaining this invention.

### Preparation Example 1

### 2-Benzylamino-2-methylthio-1,1-ethylenedicarbonitrile

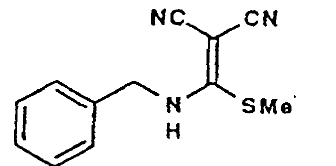

To a solution of malononitrile (1.21 g, 18.3 mmol) in DMF (40 ml) was added under ice-cooling sodium hydride (0.77 g, 19.2 mmol). After stirring for one hour, benzyl isothiocyanate (2.73 g, 18.3 mmol) was added dropwise. After stirring under ice-cooling for 1.5 hours, methyl iodide (2.60 g, 18.3 mmol) was added dropwise. After stirring at room temperature for 15 hours, water (400 ml) was added to the reaction mixture, which was then extracted with ethyl acetate (100 ml x 2). The organic layer was washed with a saturated aqueous solution of sodium chloride, dried over sodium sulfate and concentrated under reduced pressure. The residue was purified by silica gel column chromatography to give the title compound as a yellow oily substance (2.94 g) from the fraction from hexane-ethyl acetate (2/1). Yield=70%.
¹HNMR(CDCl₃) δ2.63(s,3H), 4.71(d,J=6Hz,2H), 6.82(bs,1H), 7.22-7.42(m,5H)
IR(film) 3204, 2190, 1727, 1638, 1561, 1408, 1235, 1045, 735cm⁻¹
MS m/z 229(M⁺)

### Preparation Example 2

### 2-Methylamino-2-methylthio-1,1-ethylenedicarbonitrile

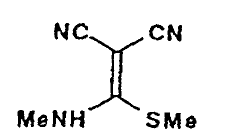

This compound was synthesized from malononitrile and methyl isothiocyanate according to the same process as in Preparation Example 1. Yield=43%.
m.p. 118-121°C (recrystallized from hexane-ethyl acetate)
¹HNMR(CDCl₃) δ2.68(s,3H), 3.22(d,J=5Hz,3H), 6.28(bs,1H)
IR(KBr) 3318, 2208, 2186, 1548, 1403, 1285cm⁻¹
MS m/z 153(M⁺)

### Preparation Example 3

### 2-Phenylamino-2-methylthio-1,1-ethylenedicarbonitrile

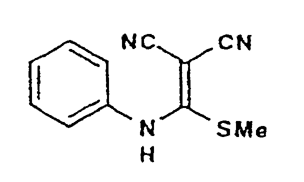

This compound was synthesized from malononitrile, phenyl isothiocyanate and methyl iodide according to the same process as in Preparation Example 1. Yield=66%.
m.p. 170-176°C (recrystallized from ethanol)
¹HNMR(CDCl₃) δ2.29(s,3H), 7.26-7.29(m,2H), 7.31-7.36(m,1H), 7.41-7.46(m,2H), 7.86(bs,1H)
IR(KBr) 3292, 2208, 2198, 2184, 1597, 1526, 1494, 1451, 1265, 968, 761, 701cm⁻¹
MS m/z 215(M⁺)

### Preparation Example 4

### 2-Cyclohexylamino-2-methylthio-1,1-ethylenedicarbonitrile

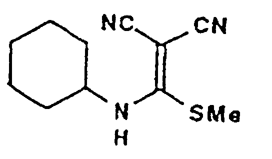

This compound was synthesized from malononitrile, cyclohexyl isothiocyanate and methyl iodide according to the same process as in Preparation Example 1. Yield=99%.
m.p. 102-103°C (recrystallized from hexane-ethyl acetate)
¹HNMR(CDCl₃) δ1.21-1.41(m,5H), 1.64-1.68(m,1H), 1.78-1.83(m,2H), 1.97-2.00(m,2H), 2.68(s,3H), 3.86-3.94(m,1H), 5.99(bs,1H)
IR(KBr) 3252, 2936, 2210, 2192, 1568, 1562, 1422, 1357, 732 cm⁻¹
MS m/z 221(M⁺)

### Preparation Example 5

### 2-Isobutylamino-2-methylthio-1,1-ethylenedicarbonitrile

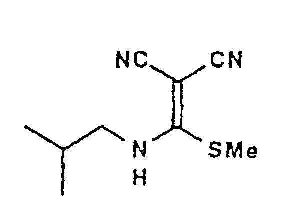

This compound was synthesized as a yellow oily substance from malononitrile, isobutyl isothiocyanate and methyl iodide according to the same process as in Preparation Example 1. Yield=91%.
¹HNMR(CDCl₃) δ0.98(d,J=7Hz,6H), 1.85-1.96(m,1H), 2.67(s,3H), 3.38(t,J=6Hz,2H), 6.49(bs,1H)
IR(film) 3242, 2962, 2210, 1562, 1373, 1271cm⁻¹
MS m/z 195(M⁺)

### Preparation Example 6

### Ethyl 3-cyclohexylamino-3-methylthio-2-cyanoacrylate

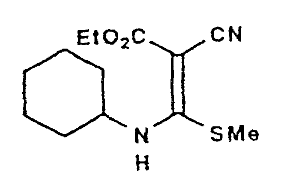

To a solution of ethyl cyanoacetate (2.07 g, 18.3 mmol) in DMF (40 ml) was added under ice-cooling sodium hydride (0.77 g, 19.2 mmol). After stirring for one hour, cyclohexyl isothiocyanate (2.59 g, 18.3 mmol) was added dropwise. After stirring under ice-cooling for 1.5 hours, methyl iodide (2.60 g, 18.3 mmol) was added dropwise. After stirring at room temperature for 15 hours, water (400 ml) was added to the reaction mixture, which was then extracted with ethyl acetate (100 ml x 2). The organic layer was washed with a saturated aqueous solution of sodium chloride, dried over sodium sulfate and concentrated under reduced pressure. The residue was purified by silica gel column chromatography to give the title compound as a yellow oily substance (4.76 g) from the fraction from hexane-ethyl acetate (4/1). Yield=97%.
¹HNMR(CDCl₃) δ1.24-1.41(m,5H), 1.32(t,J=7Hz,3H), 1.61-1.64(m,1H), 1.75-1.78(m,2H), 1.90-1.93(m,2H), 2.70(s,3H), 3.93-3.96(m,1H), 4.21(q,J=7Hz,2H), 10.10(bs,1H)
IR(KBr) 2932, 2856, 2206, 1656, 1563, 1259, 1143, 1031, 783 cm⁻¹.
MS m/z 268(M⁺)

### Preparation Example 7

### Ethyl 2-cyano-3-methylamino-3-methylthioacrylate

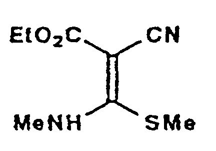

This compound was synthesized from ethyl cyanoacetate, methyl isothiocyanate and methyl iodide according to the same process as in Preparation Example 6. Yield=98%.
m.p. 87-88°C (recrystallized from hexane-ethyl acetate)
¹HNMR(CDCl₃) δ1.32(t,J=7Hz,3H), 2.68(s,3H), 3.20(d,J=5Hz,3H),4.21(q,J=7Hz,2H), 10.00(bs,1H)
IR(KBr) 2200, 1650, 1587, 1382, 1266, 1031, 775cm⁻¹
MS m/z 200(M⁺)

### Preparation Example 8

### Ethyl 2-cyano-3-phenylamino-3-methylthioacrylate

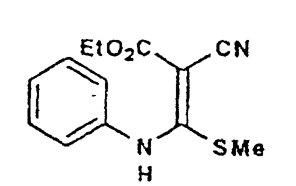

This compound was synthesized from ethyl cyanoacetate, phenyl isothiocyanate and methyl iodide according to the same process as in Preparation Example 6. Yield=68%.
m.p. 70-71°C (recrystallized from hexane-ethyl acetate)
¹HNMR(CDCl₃) δ1.35(t,J=7Hz,3H), 2.23(s,3H), 4.26(q,J=7Hz,2H),7.29-7.32(m,3H), 7.38-7.43(m,2H), 11.51(bs,1H)
IR(KBr) 2204, 1656, 1561, 1377, 1265, 1027, 767cm⁻¹
MS m/z 263(M⁺)

### Preparation Example 9

### Ethyl 2-cyano-3-isobutylamino-3-methylthioacrylate

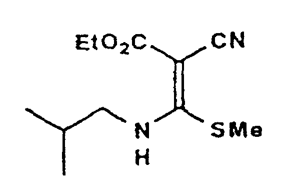

This compound was synthesized as a yellow oily substance from ethyl cyanoacetate, isobutyl isothiocyanate and methyl iodide according to the same process as in Preparation Example 6. Yield=80%.
¹HNMR(CDCl₃) δ0.98(d,J=7Hz,6H), 1.33(t,J=7Hz,3H), 1.82-1.96(m,1H), 2.67(s,3H), 3.42(t,J=7Hz,2H), 4.22(q,J=7Hz,2H), 10.17(bs,1H)
IR(KBr) 2874, 2206, 1655, 1573, 1400, 1383, 1270, 1188, 1137, 784cm⁻¹
MS m/z 242(M⁺)

### Preparation Example 10

### Ethyl 3-benzylamino-2-cyano-3-methylthioacrylate

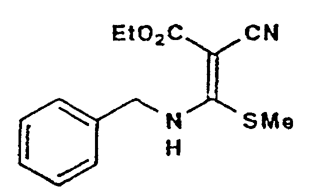

This compound was synthesized from ethyl cyanoacetate, benzyl isothiocyanate and methyl iodide according to the same process as in Preparation Example 6. Yield=61%.
m.p. 61-62°C (recrystallized from hexane-ethyl acetate)
¹HNMR(CDCl₃) δ1.31(t,J=7Hz,3H), 2.65(s,3H), 4.20(q,J=7Hz,2H), 4.77(d,J=6Hz,2H), 7.23-7.39(m,5H), 10.39(bs,1H)
IR(KBr) 3176, 2206, 1674, 1544, 1401, 1264, 1246, 1195, 742 cm⁻¹
MS m/z 276(M⁺)

### Preparation Example 11

### Ethyl 2-cyano-3-(p-methoxyphenyl)amino-3-methylthioacrylate

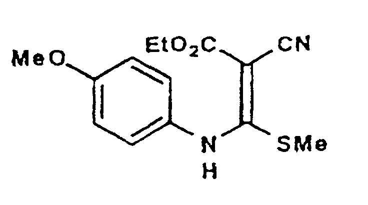

This compound was synthesized from ethyl cyanoacetate, p-methoxyphenyl isothiocyanate and methyl iodide according to the same process as in Preparation Example 6.
m.p. 83-85°C (recrystallized from hexane-ethyl acetate)
¹HNMR(CDCl₃) δ1.35(t,J=7Hz,3H), 2.27(s,3H), 3.83(s,3H), 4.26(q,J=7Hz,2H), 6.91(d,J=9Hz,2H), 7.19(d,J=9Hz,2H), 11.42(bs,1H)
IR(KBr) 3122, 2208, 1667, 1611, 1540, 1509, 1378, 1263, 1245, 1168, 1018, 781cm⁻¹
MS m/z 292(M⁺)

### Example 1

### 2-Benzylamino-2-[4-(p-fluorobenzyl)-2-morpholinylmethylamino]-1,1-ethylenedicarbonitrile

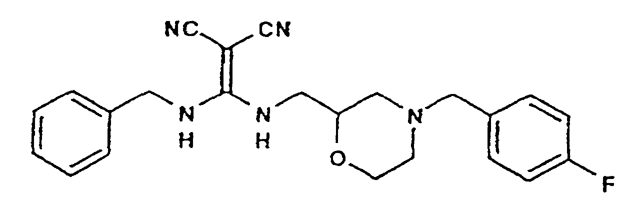

To a solution of 2-benzylamino-2-methylthio-1,1-ethylenedicarbonitrile (0.80 g, 3.49 mmol) in acetonitrile (20 ml) was added 2-aminomethyl-4-(p-fluorobenzyl)morpholine (1.17 g, 5.23 mmol) and the mixture was stirred at room temperature for 8 hours. The reaction mixture was concentrated under reduced pressure and the residue was purified by silica gel column chromatography to give the title compound as a yellow oily substance (0.90 g) from the fraction from hexane-ethyl acetate (1/1). Yield=64%.
¹HNMR(CDCl₃) δ1.88(t,J=11Hz,1H), 2.02-2.08(m,1H), 2.61(t,J=10Hz,2H), 3.16-3.23(m,1H), 3.29-3.35(m,1H), 3.42(d,J=13Hz,1H), 3.46(d,J=13Hz,1H), 3.54-3.60(m,2H), 3.67(dd,J=2Hz,11Hz,1H), 4.53-4.62(m,2H), 6.02(bs,1H), 6.98-7.04(m,2H), 7.14(bs,1H), 7.23-7.41(m,6H)
MS m/z 405(M⁺)

To a solution of the title compound (0.50 g) in ethanol (10 ml) was added under ice-cooling while stirring a solution of 4N hydrochloric acid-ethyl acetate (0.3 ml). The reaction mixture was concentrated under reduced pressure to give the corresponding hydrochloride (0.40 g) as a yellow amorphous.
m.p. 112-114°C
IR(KBr) 3300, 2202, 2176, 1562, 1515, 1229, 700cm⁻¹

### Example 2

### 2-Cyclohexylamino-2-[4-(p-fluorobenzyl)-2-morpholinylmethylamino]-1,1-ethylenedicarbonitrile

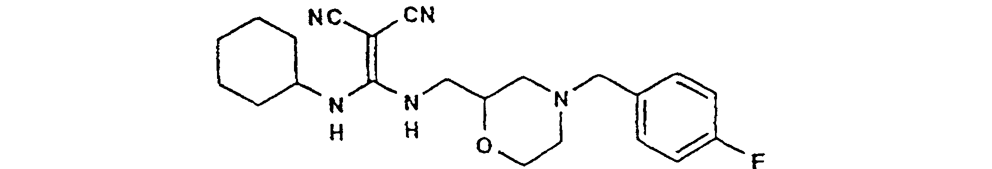

This compound was synthesized as a yellow oily substance from 2-cyclohexylamino-2-methylthio-1,1-ethylenedicarbonitrile and 2-aminomethyl-4-(p-fluorobenzyl)-morpholine according to the same procedure as in Example 1. Yield=27%.
¹HNMR(CDCl₃) δ1.21-1.28(m,4H), 1.35-1.44(m,2H), 1.59-1.72(m,4H), 1.91-2.00(m,3H), 2.14(dt,J=3Hz,11Hz,1H), 2.63-2.69(m,2H), 3.29-3.34(m,1H), 3.43(d,J=13Hz,1H), 3.47(d,J=13Hz,1H), 3.62-3.74(m,2H), 3.84-3.91(m,1H), 6.98-7.04(m,2H), 7.25-7.29(m,2H)
MS m/z 397(M⁺)

Then, the corresponding hydrochloride was prepared in a conventional manner.
m.p. 128-132°C
IR(KBr) 3240, 2930, 2200, 2174, 1515, 1228cm⁻¹

### Example 3

### 2-[4-(p-Fluorobenzyl)-2-morpholinylmethylamino]-2-phenylamino-1,1-ethylenedicarbonitrile

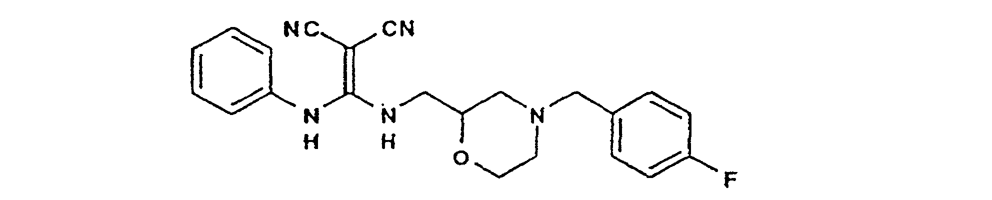

This compound was synthesized from 2-phenylamino-2-methylthio-1,1-ethylenedicarbonitrile and 2-aminomethyl-4-(p-fluorobenzyl)morpholine according to the same procedure as in Example 1. Yield=61%.
m.p. 65-67°C (a foamy solid)
¹HNMR(CDCl₃) δ1.95(t,J=11Hz,1H), 2.18(dt,J=3Hz,12Hz,1H), 2.65-2.70(m,2H), 3.25-3.31(m,2H), 3.40-3.52(m,3H), 3.67-3.75(m,2H), 3.54(d,J=10Hz,1H), 6.24(bs,1H), 7.00-7.04(m,2H), 7.10(d,J=8Hz,2H), 7.20-7.29(m,3H), 7.36-7.40(m,2H), 8.36(bs,1H)
IR(KBr) 3258, 2206, 2182, 1607, 1588, 1510, 1222, 692cm⁻¹
MS m/z 391(M⁺)

### Example 4

### 2-[4-(p-Fluorobenzyl)-2-morpholinylmethylamino]-2-methylamino-1,1-ethylenedicarbonitrile

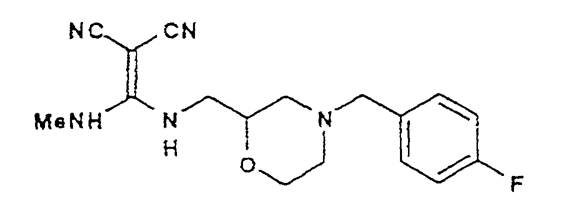

This compound was synthesized from 2-methylamino-2-methylthio-1,1-ethylenedicarbonitrile and 2-aminomethyl-4-(p-fluorobenzyl)morpholine according to the same procedure as in Example 1. Yield=87%.
m.p. 143-144°C (recrystallized from ethanol)
¹HNMR(CDCl₃) δ1.93(t,J=10Hz,1H), 2.17(dt,J=3Hz,11Hz,1H), 2.67(t,J=10Hz,2H), 3.08(d,J=5Hz,3H), 3.17-3.24(m,1H), 3.30-3.36(m,1H), 3.46(d,J=13Hz,1H), 3.51(d,J=13Hz,1H), 3.62-3.66(m,1H), 3.71(dt,J=2Hz,11Hz,1H), 3.92(dd,J=2Hz,11Hz,1H), 5.70(t,J=5Hz,1H), 6.69(bs,1H), 6.99-7.05(m,2H), 7.23-7.30(m,2H)
IR(KBr) 3294, 2870, 2204, 2174, 1603, 1586, 1509, 1221, 1058, 845cm⁻¹
MS m/z 329(M⁺)

### Example 5

### 2-[4-(p-Fluorobenzyl)-2-morpholinylmethylamino]-2-isobutylamino-1,1-ethylenedicarbonitrile

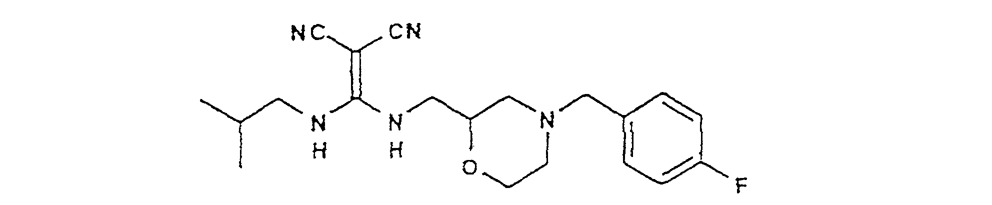

This compound was synthesized as a yellow oily substance from 2-isobutylamino-2-methylthio-1,1-ethylenedicarbonitrile and 2-aminomethyl-4-(p-fluorobenzyl)-morpholine according to the same procedure as in Example 1. Yield=48%.
¹HNMR(CDCl₃)δ0.97 (d,J=7Hz,6H), 1.82-1.92(m,1H), 1.95(t,J=11Hz,1H), 2.15(dt,J=3Hz,12Hz,1H), 2.67(d,J=12Hz,2H), 3.17-3.35(m,4H),3.47(d,J=13Hz,1H), 3.51(d,J=13Hz,1H), 3.62-3.66(m,1H), 3.71(dt,J=2Hz,11Hz,1H), 3.88(dd,J=2Hz,11Hz,1H), 5.96(t,J=6Hz,1H), 7.00-7.05(m,2H), 7.26-7.29(m,2H)
IR(KBr) 3310, 2960, 2202, 2176, 1575, 1516, 1230, 1092, 845cm⁻¹
MS m/z 371(M⁺)

Then, the corresponding hydrochloride was prepared in a conventional manner.
m.p. 110-114°C

### Example 6

### Ethyl 3-benzylamino-3-[4-(p-fluorobenzyl)-2-morpholinylmethylamino]-2-cyanoacrylate

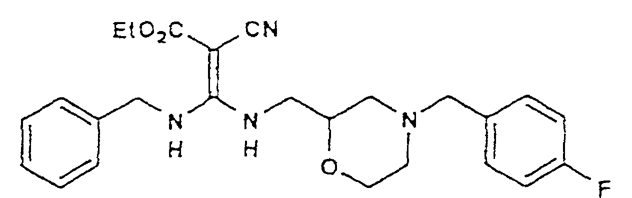

To a solution of ethyl 3-benzylamino-3-methylthio-2-cyanoacrylate (1.07 g, 3.87 mmol) in acetonitrile (10 ml) was added 2-aminomethyl-4-(p-fluorobenzyl)morpholine (0.90 g, 4.02 mmol) and the mixture was stirred at room temperature for 10 hours. The reaction mixture was concentrated under reduced pressure and the residue was purified by silica gel column chromatography to give the title compound as white crystals (0.70 g) from the fraction from hexane-ethyl acetate (1/1). Yield=40%.
m.p. 107-108°C (recrystallized from hexane-ethyl acetate)
¹HNMR(CDCl₃) δ1.30(t,J=7Hz,3H), 1.86(t,J=11Hz,1H), 2.08(dd,J=3Hz, 12Hz,1H), 2.59(d,J=11Hz,2H), 3.27(bs,1H), 3.37-3.46(br,1H),3.38(d,J=13Hz,1H), 3.45(d,J=13Hz,1H), 3.53-3.59(m,2H), 3.70(d,J=11Hz,1H), 4.18(q,J=7Hz,2H), 4.58(bs,2H), 6.97-7.02(m,2H), 7.22-7.39(m,7H)
IR(KBr) 3220, 2192, 1657, 1604, 1515, 1288, 1228, 1123, 1096, 775cm⁻¹
MS m/z 452(M⁺)

Then, the corresponding hydrochloride was prepared in a conventional manner.
m.p. 103-107°C

### Example 7

### Ethyl 3-[4-(p-fluorobenzyl)-2-morpholinylmethylamino]-3-phenylamino-2-cyanoacrylate

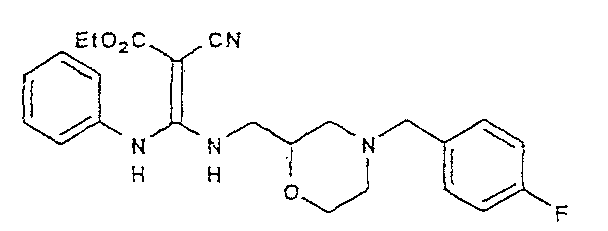

This compound was synthesized from ethyl 3-phenylamino-3-methylthio-2-cyanoacrylate and 2-aminomethyl-4-(p-fluorobenzyl)morpholine according to the same procedure as in Example 6. Yield=66%.
m.p. 114-116°C
¹HNMR(CD₃OD) δ1.26(bs,3H), 1.89(t,J=11Hz,1H), 2.19(t,J=11Hz,1H), 2.65(m,2H), 3.15-3.24(m,2H), 3.45(d,J=13Hz,1H), 3.50(d,J=13Hz,1H), 3.66(bs,2H), 3.91(d,J=11Hz,1H), 4.14(bs,2H), 7.02-7.33(m,9H)
IR(KBr) 3168, 2482, 2200, 1654, 1612, 1516, 1455, 1285, 1113, 1096, 771, 751, 696cm⁻¹
MS m/z 438(M⁺)

Then, the corresponding hydrochloride was prepared in a conventional manner.
m.p. 174-179°C.

### Example 8

### Ethyl 3-[4-(p-fluorobenzyl)-2-morpholinylmethylamino]-3-isobutylamino-2-cyanoacrylate

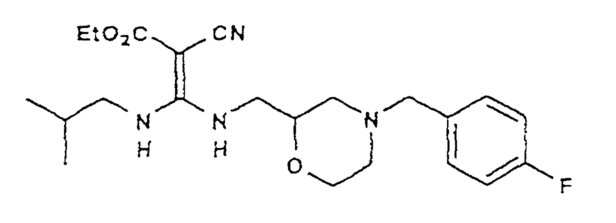

This compound was synthesized as a yellow oily substance from ethyl 3-isobutylamino-3-methylthio-2-cyanoacrylate and 2-aminomethyl-4-(p-fluorobenzyl)morpholine according to the same procedure as in Example 6. Yield=22%.
¹HNMR(CDCl₃) δ0.98(d,J=7Hz,6H), 1.29(t,J=7Hz,3H), 1.82-1.90(m,1H), 1.96(t,J=11Hz,1H), 2.17(dt,J=3Hz,12Hz,1H), 2.66(d,J=11Hz,2H), 3.15-3.42(m,4H), 3.43(d,J=13Hz,1H), 3.50(d,J=13Hz,1H), 3.64-3.73(m,2H), 3.90(dd,J=2Hz,11Hz,1H), 4.16(q,J=7Hz,2H), 6.98-7.03(m,2H), 7.25-7.29(m,2H)
IR(film) 3254, 2960, 2190, 1656, 1605, 1569, 1517, 1288, 1232,1112, 776cm⁻¹
MS m/z 418(M⁺)

Then, the corresponding hydrochloride was prepared in a conventional manner.
m.p. 133-136°C

### Example 9

### Ethyl 3-cyclohexylamino-3-[4-(p-fluorobenzyl)-2-morpholinylmethylamino]-2-cyanoacrylate

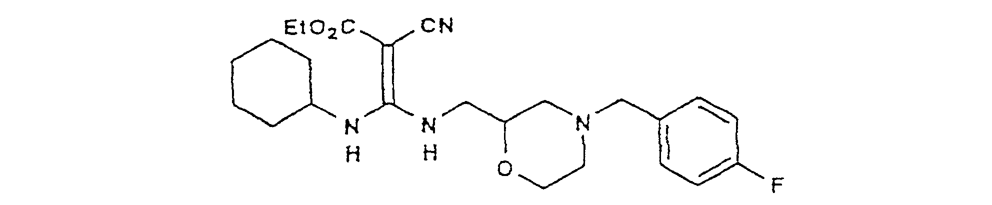

This compound was synthesized as a yellow oily substance from ethyl 3-cyclohexylamino-3-methylthio-2-cyanoacrylate and 2-aminomethyl-4-(p-fluorobenzyl)-morpholine according to the same procedure as in Example 6. Yield=10%.
¹HNMR(CDCl₃) δ1.22-1.31(m,6H), 1.28(t,J=7Hz,3H), 1.35-1.44(m,2H), 1.58-1.61(m,1H), 1.70-1.73(m,2H), 1.98-1.93(m,3H), 2.17(dt,J=3Hz,11Hz,1H), 2.66(d,J=11Hz,2H), 3.27(bs,1H), 3.40(bs,1H), 3.44(d,J=13Hz,1H), 3.50(d,J=13Hz,1H), 3.67-3.72(m,2H), 3.90(dd,J=2Hz,11Hz,1H), 4.16(q,J=7Hz,2H), 6.98-7.03(m,2H), 7.25-7.29(m,2H)
IR(film) 3228, 2856, 2190, 1656, 1605, 1515, 1287, 1228, 1124, 1094, 77cm⁻¹
MS m/z 444(M⁺)

Then, the corresponding hydrochloride was prepared in a conventional manner.
m.p. 105-113°C

### Example 10

### Ethyl 3-[4-(p-fluorobenzyl)-2-morpholinylmethylamino]-3-(p-methoxybenzyl)amino-2-cyanoacrylate

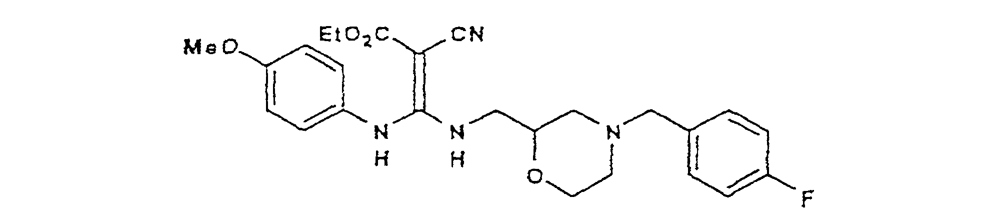

This compound was synthesized from ethyl 3-(p-methoxyphenyl)amino-3-methylthio-2-cyanoacrylate and 2-aminomethyl-4-(p-fluorobenzyl)morpholine according to the same procedure as in Example 6. Yield=71%.
m.p. 131-133°C
¹HNMR(DMSO-d₆) δ1.18(t,J=7Hz,3H), 1.79(t,J=11Hz,1H), 2.08(dt,J=3Hz,11Hz,1H), 2.59(d,J=11Hz,2H), 3.12-3.27(m,2H), 3.34(s,3H), 3.39(d,J=13Hz,1H), 3.47(d,J=13Hz,1H), 3.50-3.57(m,2H), 3.82(d,J=11Hz,1H), 4.05(q,J=7Hz,2H), 6.88(d,J=9Hz,2H), 7.00(d,J=9Hz,2H), 7.10-7.14(m,2H), 7.29-7.33(m,2H)
IR(KBr) 3240, 2932, 2196, 1656, 1615, 1513, 1250, 1239, 1220, 1117, 1142, 825cm⁻¹
MS m/z 468(M⁺)

Then, the corresponding hydrochloride was prepared in a conventional manner.
m.p. 125-131°C

### Example 11

### Ethyl 3-[4-(p-fluorobenzyl)-2-morpholinylmethylamino]-3-methylamino-2-cyanoacrylate

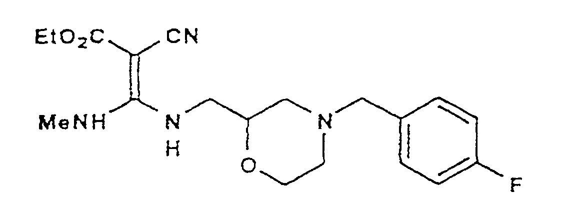

This compound was synthesized as a yellow oily substance from ethyl 3-methylamino-3-methylthio-2-cyanoacrylate and 2-aminomethyl-4-(p-fluorobenzyl)morpholine according to the same procedure as in Example 6. Yield=53%.
¹HNMR(CDCl₃) δ1.30(t,J=7Hz,3H), 1.95(1,J=11Hz,1H), 2.17(dt,J=3Hz,11Hz,1H), 2.65(d,J=11Hz,2H), 3.06(bs,3H), 3.23-3.55(m,2H), 3.43(d,J=13Hz,1H), 3.50(d,J=13Hz,1H), 3.66-3.73(m,2H), 3.91(dd,J=2Hz,11Hz,1H), 4.17(q,J=7Hz,2H), 6.99-7.03(m,2H), 7.29-7.50(m,2H)
IR(film) 3232, 2186, 1738cm⁻¹
MS m/z 376(M⁺)

### Example 12

### Ethyl 3-cyclohexylamino-3-[7-(p-fluorophenyl)-6-aza-3-oxaheptylamino]-2-cyanoacrylate

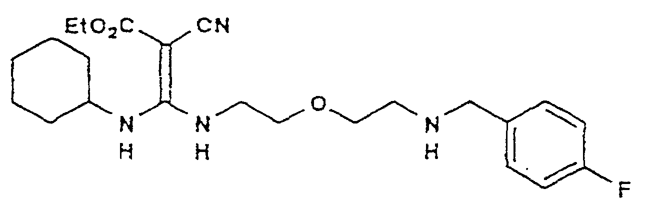

### a) Synthesis of ethyl 3-[6-(tert-butyloxycarbonyl)-7-(p-fluorophenyl)-6-aza-3-oxaheptylmethyl]-3-cyclohexylamino-2-cyanoacrylate

To a solution of ethyl 3-cyclohexylamino-3-methylthio-2-cyanoacrylate (1.50 g, 5.60 mmol) in acetonitrile (10 ml) was added tert-butoxy N-(5-amino-3-oxapentyl)-N-(p-fluorobenzyl)carbamate (3.10 g, 11.2 mmol) and the mixture was stirred at room temperature for 18 hours. The reaction mixture was concentrated and the residue was purified by silica gel column chromatography to give the title compound as a pale yellow oily substance (1.72 g) from the fraction from hexane-ethyl acetate (2/1). Yield=58%.
¹HNMR(CDCl₃) δ1.16-1.44(m,4H), 1.29(t,J=7Hz,3H), 1.44(bs,9H),1.58-1.64(m,2H), 1.70-1.72(m,2H), 1.94-2.04(m,2H), 3.33-3.55(m,8H), 4.16(q,J=7Hz,2H), 4.43(bs,2H), 6.99-7.04(m,2H), 7.19(bs, 2H)
IR(film) 3308, 2932, 2193, 1607, 1513cm⁻¹

### b) Synthesis of ethyl 3-cyclohexylamino-3-[7-(p-fluorophenyl)-6-aza-3-oxaheptylamino]-2-cyanoacrylate

To a solution of ethyl 3-[6-(tert-butyloxycarbonyl)-7-(p-fluorophenyl)-6-aza-3-oxaheptylmethyl]-3-cyclohexylamino-2-cyanoacrylate (1.72 g, 3.23 mmol) in methanol (15 ml) was added a 4N hydrochloric acid-ethyl acetate solution (2.5 ml) and the mixture was stirred at room temperature for 14 hours. The reaction mixture was concentrated and water (50 ml) was added to the residue. The mixture was adjusted to pH 12 with an aqueous solution of sodium hydroxide and extracted with chloroform (50 ml x 3). The organic layer was dried over sodium sulfate and the solvent was distilled off under reduced pressure. The residue was purified by silica gel column chromatography to give the title compound as a yellow oily substance (1.06 g) from the fraction from chloroform-methanol-aqueous ammonia (30/1/0.05). Yield=76%.
¹HNMR(CDCl₃) δ1.23-1.40(m,8H), 1.58-1.61(m,1H), 1.70-1.75(m,4H), 1.95-1.98(m,2H), 2.81(t,J=5Hz,2H), 3.58-3.65(m,5H), 3.78(s,2H), 4.13(q,J=7Hz,2H), 6.99-7.03(m,2H), 7.26-7.31(m,2H)
IR(film) 3222, 2856, 2190, 1649, 1223, 778cm⁻¹
MS m/z 432(M⁺)

Then, the corresponding hydrochloride was prepared in a conventional manner (a colorless and high viscous oil).

### Example 13

### 2-(p-Fluorobenzylamino)-2-[4-(p-fluorobenzyl)-2-morpholinylmethylamino]-1,1-ethylenedicarbonitrile

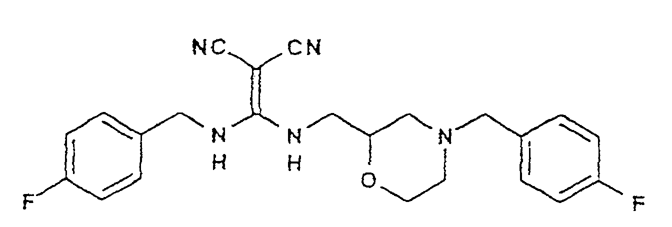

### a) 2-[4-(p-Fluorobenzyl)-2-morpholinylmethylamino]-2-methylthio-1,1-ethylenedicarbonitrile

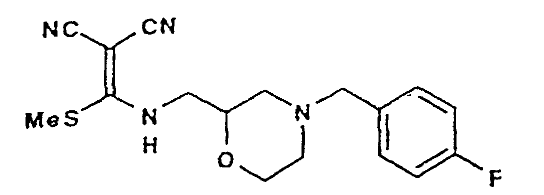

To a solution of 2-aminomethyl-4-(p-fluorobenzyl)-morpholine (500 mg, 2.23 mmol) in acetonitrile (10 ml) was added 2,2-dimethylthio-1,1-ethylenedicarbonitrile (417 mg, 2.46 mmol) and the mixture was stirred at room temperature for 2.5 hours. The reaction mixture was concentrated under reduced pressure and the residue was subjected to silica gel column chromatography to give the title compound (650 mg) from the fraction from ethyl acetate-hexane (2/1). Yield=84%.
¹HNMR(CDCl₃) δ1.92(t,J=10Hz,1H), 2.21(dt,J=3Hz,11Hz,1H), 2.52-2.76(m,2H), 2.62(s,3H), 3.35-3.57(m,3H), 3.58-3.79(m,3H), 3.82-3.97(s,1H), 6.30-6.53(br,1H), 7.02(t,J=8Hz,2H), 7.15-7.36(m,2H)

### b) 2-(p-Fluorobenzylamino)-2-[4-(p-fluorobenzyl)-2-morpholinylmethylamino]-1,1-ethylenedicarbonitrile

To a solution of 2-[4-(p-fluorobenzyl)-2-morpholinylmethylamino]-2-methylthio-1,1-ethylenedicarbonitrile (650 mg, 1.88 mmol) in acetonitrile (7 ml) was added p-fluorobenzylamine (0.24 ml, 2.1 mmol), and the mixture was heated under reflux for 13 hours. The reaction mixture was concentrated under reduced pressure to distill off the solvent and the residue was chromatographed using silica gel column to give the title compound (760 mg) as a pale yellow oily substance from the fraction from ethyl acetate-hexane (2/1). Yield=96%.
¹HNMR(CDCl₃) δ1.87(t,J=11.23Hz,1H), 1.98-2.12(m,1H), 2.51-2.68(m,2H), 3.11-3.23(m,1H), 3.23-3.36(m,1H), 3.42(d,J=12.7Hz,1H), 3.48(d,J=13.19Hz,1H), 3.51-3.73(m,3H), 4.56(d,J=4.88Hz,2H),5.62-5.78(brm,1H), 6.90-7.13(m,4H), 7.18-7.32(m,4H)
IR(film) 3300, 2205, 2180, 1600, 1580, 1505cm⁻¹

### Example 14

### Ethyl 2-cyano-3-[endo-9-(p-fluorobenzyl)-9-aza-3-oxabicyclo[3.3.1]non-7-ylamino]-3-methylaminoacrylate

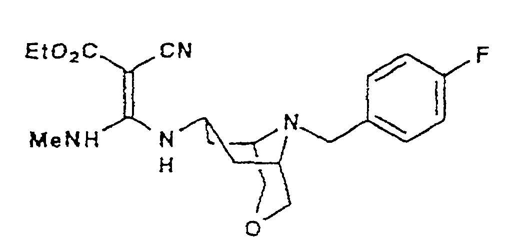

To a solution of endo-7-amino-9-(p-fluorobenzyl)-9-aza-3-oxabicyclo[3.3.1]nonane (280 mg, 0.979 mmol) in acetonitrile (5 ml) was added ethyl 2-cyano-3-methylamino-3-methylthioacrylate (249 mg, 1.27 mmol) and the mixture was stirred at room temperature for 1.5 hours and then heated under reflux for 8 hours. The reaction mixture was concentrated under reduced pressure and the residue was subjected to silica gel column chromatography to give the title compound (220 mg) from the fraction with ethyl acetate-hexane (1/1). Yield=52%.
¹HNMR(CDCl₃) δ1.29(t,J=7Hz,3H), 1.54(d,J=16Hz,2H), 2.41-2.58(m,2H), 2.65-2.80(m,2H), 2.85(d,J=5Hz,3H), 3.79(d,J=14Hz,4H), 3.98(d,J=11Hz,2H), 4.17(q,J=7Hz,2H), 4.73(bs,1H), 6.95-7.06(m,2H), 7.29(m,2H), 7.82(bs,1H), 9.00(bs,1H)
IR(KBr) 3275, 2200, 1645cm⁻¹

### Example 15

### 2-[endo-9-(p-Fluorobenzyl)-9-aza-3-oxabicyclo[3.3.1]non-7-ylamino]-2-methylamino-1,1-ethylenedicarbonitrile

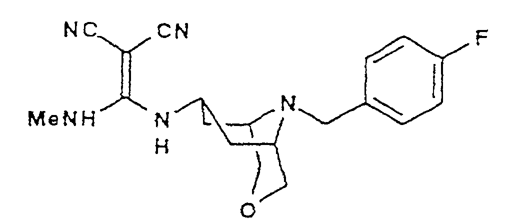

This compound was synthesized from endo-7-amino-9-(p-fluorobenzyl)-9-aza-3-oxabicyclo[3.3.1]nonane and 2-methylamino-2-methylthio-1,1-ethylenedicarbonitrile according to the same procedure as in Example 14. Yield=100%.
m.p. 226.5-228°C
¹HNMR(CDCl₃) δ1.42-1.72(m,4H), 1.40-1.60(m,2H), 2.65-2.80(br,2H), 2.86(d,J=5Hz,3H), 3.76(d,J=9Hz,2H), 3.99(d,J=11Hz,2H), 3.41-3.63(br,1H), 5.23-5.42(br,1H), 7.01(t,J=8Hz,2H), 7.18-7.47(m,2H), 8.13(d,J=11Hz,1H)
IR(film) 2200, 2175, 1560cm⁻¹

### Example 16

### 2-[endo-9-[3-(p-Fluorophenoxy)propyl]-9-aza-3-oxabicyclo-[3.3.1]non-7-ylamino]-2-methylamino-1,1-ethylenedicarbonitrile

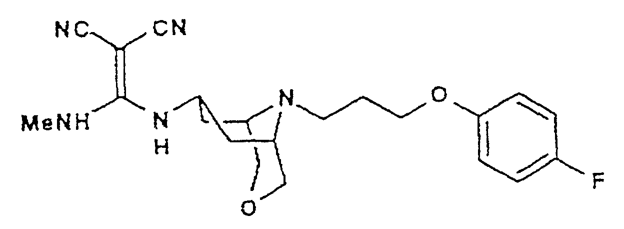

This compound was synthesized from 2-methylamino-2-methylthio-1,1-ethylenedicarbonitrile and endo-7-amino-9-[3-(p-fluorophenoxy)propyl]-9-aza-3-oxabicyclo[3.3.1]-nonane according to the same procedure as in Example 14. Yield=32%.
m.p. 195-196°C (recrystallized from hexane-ethyl acetate)
¹HNMR(CDCl₃)δ 1.52(d,J=16Hz,2H), 1.86(dd,J=6Hz,7Hz,2H), 2.43-2.50(m,2H), 2.78-2.81(m,4H), 2.85(d,J=5Hz,3H), 3.77(d,J=12Hz, 2H), 3.96(d,J=11Hz,2H), 3.99(t,J=6Hz,2H), 4.46(bs,1H), 5.51(bs,1H), 6.80-6.85(m,2H), 6.94-7.00(m,2H), 8.10(d,J=10Hz,1H)
IR(KBr) 3282, 2200, 2176, 1559, 1509, 1428, 1386, 1338, 1247, 1205, 831cm⁻¹
MS m/z 399(M⁺)

### Example 17

### N-[endo-9-(p-Fluorobenzyl)-9-aza-3-oxabicyclo[3.3.1]non-7-yl]-N'-methyl-2-nitrovinylidene-1,1-diamine

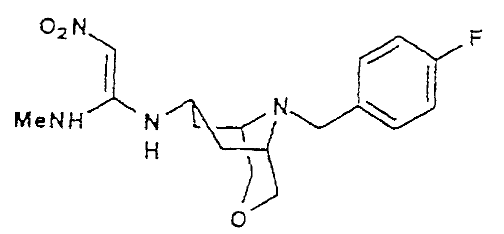

endo-7-Amino-9-aza-9-(p-fluorobenzyl)-3-oxabicyclo[3.3.1]nonane (1.55 g) and N-(1-methylthio-2-nitrovinyl)-N-methylamine (2.11 g) were heated at 80°C for 2 hours. The reaction mixture was purified by silica gel column chromatography to give the title compound (0.65 g) from the fraction from chloroform-methanol-aqueous ammonia (20/1/0.5). Yield=30%.
m.p. 184-186°C
¹HNMR(CDCl₃) δ1.48(d,J=15Hz,2H), 2.39-2.45(m,2H), 2.73(bs, 2H), 2.89(d,J=5Hz,3H), 3.73(d,J=11Hz,2H), 3.80(s,2H), 3.81-3.87(m,2H), 4.01(d,J=11Hz,2H), 6.66(s,1H), 6.99-7.05(m,2H), 7.30- 7.34(m,2H), 7.85(d,J=10Hz,1H), 10.23(bs,1H)
IR(KBr) 2940, 1676, 1570, 1516, 1229, 1072, 924cm⁻¹

To a solution of the title compound (0.63 g) in chloroform (10 ml) was added uncer ice-cooling a 4N hydrochloric acid-ethyl acetate solution (0.5 ml). The crystal thus separated out was recovered by filtration and dried under reduced pressure to give the corresponding hydrochloride (0.64 g).
m.p. 180°C (dec.)

### Example 18

### 1-[4-(p-Fluorobenzyl)-2-morpholinylmethyl]-3-phenylurea

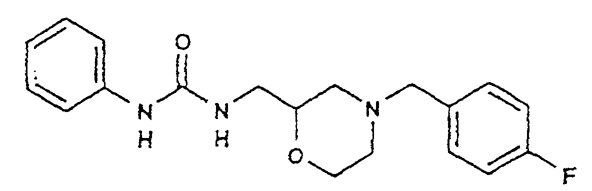

To a solution of 2-aminomethyl-4-(p-fluorobenzyl)-morpholine (1.0 g, 4.5 mmol) in dimethylformamide (10 ml) was added under ice-cooling phenyl isocyanate (0.53 ml, 4.9 mmol) and the mixture was stirred for 30 minutes- To the reaction mixture was added purified water (20 ml) and the crystals thus separated out were then recovered by filtration to give the title compound (1.1 g). Yield=72%.
¹HNMR(CDCl₃) δ1.93(t,J=11Hz,1H,), 2.13(dt,J=3Hz,12Hz,1H,), 2.63(d,J=11Hz,1H,), 2.70(d,J=11Hz,1H), 3.10-3.22(m,1H), 3.38- 3.50(m,1H), 3.44(s,2H), 3.58-3.70(m,2H), 3.84(dd,J=1Hz,11Hz,1H),5.10-5.23(br,1H), 6.70-6.89(br,1H), 6.94-7.14(m,3H), 7.19-7.39(m,6H)
IR(KBr) 3350, 1648, 1562, 1500, 1220cm⁻¹

Then, the corresponding hydrochloride was prepared in a conventional manner.
m.p. 183-187°C

### Example 19

### 1-[4-(p-Fluorobenzyl)-2-morpholinylmethyl]-3-methylurea

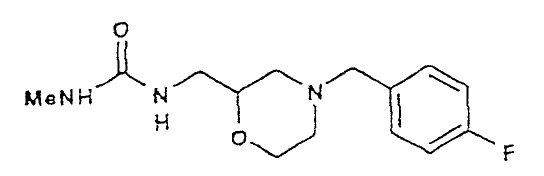

This compound was synthesized from 2-aminomethyl-4-(p-fluorobenzyl)morpholine and methyl isocyanate according to the same procedure as in Example 18. Yield=65%.
m.p. 164-166°C
¹HNMR(CDCl₃) δ1.90(t,J=11Hz,1H), 2.12(dt,J=3Hz,11Hz,1H), 2.62(dd,J=1Hz,11Hz,1H), 2.69(dd,J=2Hz,11Hz,1H), 2.76(d,J=5Hz,3H), 3.01-3.13(m,1H), 3.31-3.49(m,1H), 3.44(s,2H), 3.52-3.61(m,2H), 3.75-3.90(m,1H), 4.50-4.82(br,2H), 7.00(t,J=9Hz,2H), 7.18-7.34(m,2H)
IR(film) 3320, 1638, 1590cm⁻¹

### Example 20

### 1-[endo-9-(p-Fluorobenzyl)-9-aza-3-oxabicyclo[3.3.1]non-7-yl]-3-methylurea

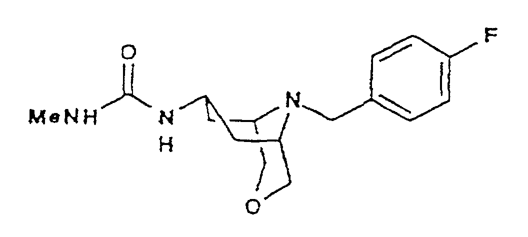

To a solution of endo-7-amino-9-(p-fluorobenzyl)-9-aza-3-oxabicyclo[3.3.1]nonane (670 mg, 2.34 mmol) in toluene (7 ml) was added under ice-cooling methyl isocyanate (0.15 ml, 2.6 mmol) and the mixture was stirred for 30 minutes. To the reaction mixture was added ethyl acetate (30 ml) and the mixture was washed with a 10% aqueous sodium hydroxide solution (20 ml), dried over potassiun carbonate and the solvent was distilled off under reduced pressure. The residue was subjected to silica gel column chromatography to give the title compound (690 mg) from the fraction from methanol-chloroform (1/9). Yield=86%.
m.p. 167-169°C
¹HNMR(CDCl₃) δ1.41(d,J=15Hz,2H), 2.31-2.44(m,2H), 2.62(bs, 2H), 2.74(d,J=5Hz,3H), 3.72(d,J=11Hz,2H), 3.78(s,2H), 3.94(d,J=11Hz,2H), 4.29-4.40(m,1H), 4.44-4.61(m,1H), 6.93-7.12(m,2H), 7.26-7.39(m,2H)
IR(film) 3375, 1645cm⁻¹

### Example 21

Acetylcholine-release promoting action of the present compounds in the gastrointestinal tract was investigated according to the following procedure. A longitudinal muscle sample (including myenteric plexus) was prepared from the ileum excised from guinea pig and suspended in Magnus' tube. This sample was perfused in a physiological salt solution and stimulated by the electric current via platinum electrodes. Acetylcholine was released from the myenteric plexus of the sample by this stimulation and the longitudinal muscle was observed to contract. This contraction was isometrically recorded. Accordingly, the drug capable of accelerating the release of acetylcholine could enhance the contraction caused by electric stimulation only. Evaluation of the compounds was represented in terms of increase ratio in contraction by electrical stimulation. The tested compounds are shown in terms of the numbers of the corresponding Examples. The results are shown below.

| Contraction increase rate (%) | | |
|---|---|---|
| Example No. | 10⁻⁷M | 10⁻⁵M |
| 1 | 3.9 | 12.3 |
| 2 | | 5.3 |
| 5 | | 4.9 |
| 6 | 3.0 | 20.3 |
| 7 | 0.7 | |
| 8 | 8.5 | 32.4 |
| 9 | 8.7 | 31.9 |
| 10 | 9.1 | 11.8 |
| 12 | 0.8 | 7.9 |
| 13 | 4.9 | 15.0 |
| 15 | 3.9 | |
| 16 | 3.1 | |
| 17 | 3.2 | 5.7 |
| 19 | 9.8 | |
| 20 | 4.2 | |

Finally, illustrative examples of the pharmaceutical compositions which comprises as an active ingredient the present compound are given below by way of the following Examples.

### Example 22

### (Formulation Example 1)

### Tablets (one tablet)

| | |
|---|---|
| The compound of Example 8 | 1 mg |
| Lactose | 70 mg |
| Crystalline cellulose | 20 mg |
| Corn starch | 8 mg |
| Magnesium stearate | 1 mg |
| Total | 100 mg |

All components were uniformly mixed to form a powder for direct compression. This powder was formed to tablets, each having a diameter of 6 mm and a weight of 100 mg.

### (Formulation Example 2)

### Granules (one package)

| | | |
|---|---|---|
| A | The compound of Example 9 | 1 mg |
| | Lactose | 99 mg |
| | Corn starch | 50 mg |
| | Crystalline cellulose | 50 mg |
| B | Hydroxypropylcellulose | 10 mg |
| | Ethanol | 9 mg |

After all components of the above group A were uniformly mixed, the solution of the above group B was added. The mixture was kneaded, graded by an extrusion granulation method and then dried in a drier at 50°C. The granules as dried up was sieved to a grain size of 297 µm - 1460 µm, thereby forming granules. One package comprised 200 mg.

### (Formulation Example 3)

### Syrups

| | |
|---|---|
| The compound of Example 1 | 0.100 g |
| Sucrose | 30.000 g |
| D-Sorbitol 70w/v% | 25.900 g |
| Ethyl para-hydroxybenzoate | 0.030 g |
| Propyl para-hydroxybenzoate | 0.015 g |
| Flavors | 0.200 g |
| Glycerol | 0.150 g |
| 96% Ethanol | 0.500 g |
| Distilled water | to make up a total volume to 100 ml |

The sucrose, D-sorbitol, ethyl para-hydroxybenzoate, propyl para-hydroxybenzoate and compound of Example 1 were dissolved in 60 g of hot water. After cooling, a solution of the flavors in the glycerol and ethanol was added. Then, the water was added to the resulting mixture to make up to a 100 ml volume.

### Industrial Applicability

The diaminomethylidene derivatives (I) or pharmacologically acceptable salts thereof as provided by the present invention can be applied for the therapy of digestive tract disorders derived from chronic gastritis, diabetes mellitus, post-gastrectomy and peptic ulcer and digestive tract diseases including reflux esophagitis, irritable bowel syndrome and spurious ileus, thus being useful as a gastrointestinal prokinetic agent.

## Claims

1. A diaminomethylidene derivative represented by formula (1) 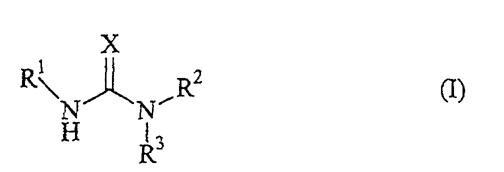 wherein
R¹ is a hydrogen atom, a C₁-C₆ alkyl group, a C₃-C₆ cycloalkyl group, a C₃-C₆ cycloalkyl C₁-C₄ alkyl group, an aryl group or an aryl C₁-C₄ alkyl group, in which the aryl moiety of the aryl group or aryl C₁-C₄ alkyl group may be optionally substituted with a halogen atom, a C₁-C₆ alkyl group, a halo C₁-C₆ alkyl group, a C₁-C₆ alkoxy group, a C₁-C₆ alkoxycarbonyl group, a phenyl group or an amino group;
X is O, S, CHNO₂, C(COOR⁴)₂, C(COOR⁴) CN or C(CN)₂, in which R⁴ is a C₁-C₆ alkyl group, a C₃-C₆ cycloalkyl group, an aryl group, or an aryl C₁-C₄ alkyl group; and
R² is a group of the following general formula (II) or (III) 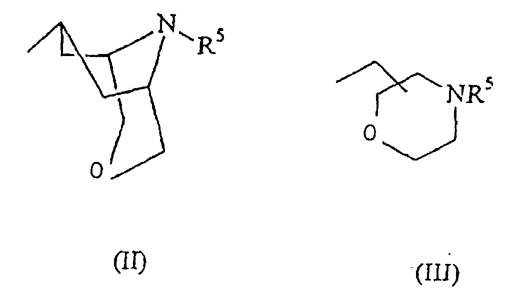 ; or
X is CHNO₂, C(COOR⁴)₂, C(COOR⁴) CN or C(CN)₂, in which R⁴ is as defined above; and
R² is a group of the following general formula (IV) 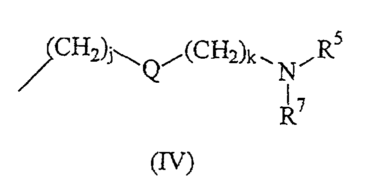 (wherein j is 0-3, k is 0-3, and the sum of j and k is 1-6, and Q is O);
R³ is a hydrogen atom, a C₁-C₆ alkyl group or a C₁-C₆ alkoxy C₂-C₆ alkyl group;
R⁵ is an aryl C₁-C₄ alkyl group, a heteroaryl C₁-C₄ alkyl group, an aryloxy C₂-C₆ alkyl group, or a pyrrolidinylcarbonyl C₁-C₄ alkyl group, in which the aryl moiety of the said groups may be optionally substituted with a halogen atom, a C₁-C₆ alkyl group, a halo C₁-C₆ alkyl group, a C₁-C₆ alkoxy group, a C₁-C₆ alkoxycarbonyl group, a phenyl group or an amino group; and
R⁷ is a hydrogen atom, a C₁-C₆ alkoxy C₂-C₄ alkyl group;
or a C₁-C₄ alkyl group; or a pharmacologically acceptable salt thereof.

2. A compound according to claim 1 where R¹ is a methyl, isobutyl, cyclohexyl, phenyl, p-methoxyphenyl, benzyl or p-fluorobenzyl group.

3. A compound according to claim 1 or 2 wherein R³ is a hydrogen atom.

4. A compound according to any preceding claim wherein R⁵ is a p-fluorobenzyl or a 3-(p-fluorophenoxy) propyl group.

5. A compound according to claim 1 in which
R¹ is a methyl, isobutyl, cyclohexyl, phenyl or benzyl group,
X is C(COOC₂H₅)CN or C(CN)₂,
R² is p-fluorobenzyl-2-morpholinyl methyl, and
R³ is a hydrogen atom.

6. A compound according to claim 1 wherein
R¹ is a p-methoxyphenyl group,
X is a C(COOC₂H₅)CN group,
R² is p-fluorobenzyl-2-morpholinyl methyl, and
R³ is a hydrogen atom.

7. A compound according to claim 1 wherein
R' is a p-fluorobenzyl group,
X is a C(CN)₂ group,
R² is p-fluorobenzyl-2-morpholinyl methyl, and
R³ is a hydrogen atom.

8. A compound according to claim 1 wherein
R' is a methyl group,
X is O, C(COOC₂H₅)CN, CHNO₂ or C(CN)₂ group,
R² is a group of the fomnula (II), wherein R⁵ is a p-fluorobenzyl group, and
R³ is a hydrogen atom.

9. A compound according to claim 1 wherein
R¹ is a cyclohexyl group,
X is a C(COOC₂H₅)CN group,
R² is a 7-(p-fluorophenyl) - 6 aza-3oxaheptyl group and
R³ is a hydrogen atom.

10. A compound according to claim 1 wherein
R' is a methyl group,
X is C(CN)₂,
R² is a compound of formula (II) wherein R⁵ is a 3-(p-fluorophenoxy)propyl group, and
R³ is a hydrogen atom.

11. A compound according to claim 1 wherein
R' is a methyl or phenyl group,
X is O,
R² is a group of the formula (III),
R⁵ is a p-fluorobenzyl group, and
R³ is a hydrogen atom.

12. A gastrointestinal prokinetic composition which comprises, as active ingredient, a compound as claimed in any preceding claim, optionally, in admixture with a pharmaceutically acceptable additive.

13. A compound as claimed in any one of claims I to 11 for use in the therapy of digestive tract disorders derived from chronic gastritis, diabetes mellitus, post-gastrectomy or peptic ulcer or digestive tract diseases.

14. A compound as claimed in any one of claims I to 11 for use in the therapy of reflux esophagitis, irritable bowel syndrome or spurious ileus.

15. Use of a compound as claimed in any one of claims 1 to 11 in the manufacture of a medicament for use in the treatment of digestive tract disorders derived from chronic gastritis, diabetes mellitus, post-gastrectomy or peptic ulcer or digestive tract diseases.

16. Use of a compound as claimed in any one of claims 1 to 11 in the manufacture of a medicament for use in the treatment of reflux esophagitis, irritable bowel syndrome or spurious ileus.

## Patentansprüche

1. Diaminomethyliden-Derivat der Formel (I) 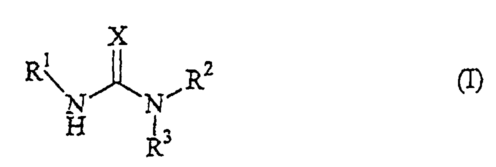 worin
R¹ ein Wasserstoffatom, eine C₁-C₆-Alkylgruppe, eine C₃-C₆-Cycloalkylgruppe, eine C₃-C₆-Cycloalkyl-C₁-C₄-alkylgruppe, eine Arylgruppe oder eine Aryl-C₁-C₄-alkylgruppe bedeutet, wobei der Arylrest der Arylgruppe oder der Aryl-C₁-C₄-alkylgruppe gegebenenfalls durch ein Halogenatom, eine C₁-C₆-Alkylgruppe, eine Halogen-C₁-C₆-alkylgruppe, eine C₁-C₆-Alkoxygruppe, eine C₁-C₆-Alkoxycarbonylgruppe, eine Phenylgruppe oder eine Aminogruppe substituiert ist;
X die Bedeutungen O, S, CHNO₂, C(COOR⁴)₂, C(COOR⁴)CN oder C(CN)₂ hat, wobei R⁴ eine C₁-C₆-Alkylgruppe, eine C₃-C₆-Cycloalkylgruppe, eine Arylgruppe oder eine Aryl-C₁-C₄-alkylgruppe bedeutet; und
R² eine Gruppe der folgenden allgemeinen Formeln (II) oder (III) 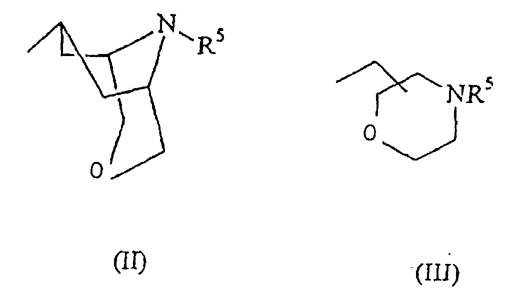 bedeutet; oder
X die Bedeutungen CHNO₂, C(COOR⁴)₂, C(COOR⁴)CN oder C(CN)₂ hat, worin R⁴ die vorstehend definierte Bedeutung hat; und
R² eine Gruppe der folgenden allgemeinen Formel (IV) 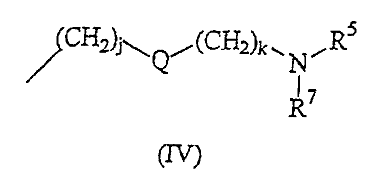 bedeutet,
(worin j einen Wert von 0-3 hat, k einen Wert von 0-3 hat und die Summe von j und k 1-6 ist und Q die Bedeutung O hat);
R³ ein Wasserstoffatom, eine C₁-C₆-Alkylgruppe oder eine C₁-C₆-Alkoxy-C₂-C₆-alkylgruppe bedeutet;
R⁵ eine Aryl-C₁-C₄-alkylgruppe, eine Heteroaryl-C₁-C₄-alkylgruppe, eine Aryloxy-C₂-C₆-alkylgruppe oder eine Pyrrolidinylcarbonyl-C₁-C₄alkylgruppe bedeutet, wobei der Arylrest dieser Gruppen gegebenenfalls durch ein Halogenatom, eine C₁-C₆-Alkylgruppe, eine Halogen-C₁-C₆alkylgruppe, eine C₁-C₆-Alkoxygruppe, eine C₁-C₆-Alkoxycarbonylgruppe, eine Phenylgruppe oder eine Aminogruppe substituiert ist;
R⁷ ein Wasserstoffatom, eine C₁-C₆-Alkoxy-C₂-C₄-alkylgruppe oder eine C₁-C₄-Alkylgruppe bedeutet;
oder ein pharmakologisch verträgliches Salz davon.

2. Verbindung nach Anspruch 1, wobei R¹ eine Methyl-, Isobutyl-, Cyclohexyl-, Phenyl-, p-Methoxyphenyl-, Benzyl oder p-Fluorbenzylgruppe bedeutet.

3. Verbindung nach Anspruch 1 oder 2, wobei R³ ein Wasserstoffatom bedeutet.

4. Verbindung nach einem der vorstehenden Ansprüche, wobei R⁵ eine p-Fluorbenzyl- oder 3-(p-Fluorphenoxy)-propylgruppe bedeutet.

5. Verbindung nach Anspruch 1, wobei
R¹ eine Methyl-, Isobutyl-, Cyclohexyl-, Phenyl- oder Benzylgruppe bedeutet,
X die Bedeutung C(COOC₂H₅)CN oder C(CN)₂ hat,
R² eine p-Fluorbenzyl-2-morpholinylmethylgruppe bedeutet und
R³ ein Wasserstoffatom bedeutet.

6. Verbindung nach Anspruch 1, wobei
R¹ eine p-Methoxyphenylgruppe bedeutet,
X eine C(COOC₂H₅)CN-Gruppe bedeutet,
R² eine p-Fluorbenzyl-2-morpholinylmethylgruppe bedeutet und
R³ ein Wasserstoffatom bedeutet.

7. Verbindung nach Anspruch 1, wobei
R¹ eine p-Fluorbenzylgruppe bedeutet,
X eine C(CN)₂-Gruppe bedeutet,
R² eine p-Fluorbenzyl-2-morpholinylmethylgruppe bedeutet und
R³ ein Wasserstoffatom bedeutet.

8. Verbindung nach Anspruch 1, wobei
R¹ eine Methylgruppe bedeutet,
X die Bedeutung O, C(COOC₂H₅)CN, CHNO₂ oder C(CN)₂ hat,
R² eine Gruppe der Formel (II) bedeutet, wobei R⁵ eine p-Fluorbenzylgruppe bedeutet und
R³ ein Wasserstoffatom bedeutet.

9. Verbindung nach Anspruch 1, wobei
R¹ eine Cyclohexylgruppe bedeutet,
X eine C(COOC₂H₅)CN-Gruppe bedeutet,
R² eine 7-(p-Fluorphenyl)-6-aza-3-oxaheptylgruppe bedeutet und
R³ ein Wasserstoffatom bedeutet.

10. Verbindung nach Anspruch 1, wobei
R¹ eine Methylgruppe bedeutet,
X die Bedeutung C(CN)₂ hat,
R² eine Verbindung der Formel (II) bedeutet, wobei R⁵ eine 3-(p-Fluorphenoxy)-propylgruppe bedeutet und
R³ ein Wasserstoffatom bedeutet.

11. Verbindung nach Anspruch 1, wobei
R¹ eine Methyl- oder Phenylgruppe bedeutet,
X die Bedeutung O hat,
R² eine Gruppe der Formel (III) bedeutet,
R⁵ eine p-Fluorbenzylgruppe bedeutet und
R³ ein Wasserstoffatom bedeutet.

12. Gastrointestinale prokinetische Zusammensetzung, umfassend als Wirkstoff eine Verbindung nach einem der vorstehenden Ansprüche gegebenenfalls im Gemisch mit einem pharmazeutisch vertrgäglichen Additiv.

13. Verbindung nach einem der Ansprüche 1 bis 11 zur Verwendung bei der Therapie von Störungen des Verdauungstraktes, die sich von chronischer Gastritis, Diabetes mellitus, dem Zustand nach Magenresektion, gastrointestinalem Ulcus oder Krankheiten des Verdauungstraktes ableiten.

14. Verbindung nach einem der Ansprüche 1 bis 11 zur Verwendung bei der Therapie von Rückfluss-Ösophagitis, irritablem Darmsyndrom oder falschem Darmverschluss.

15. Verwendung einer Verbindung nach einem der Ansprüche 1 bis 11, bei der Herstellung eines Arzneimittels zur Verwendung bei der Behandlung von Störungen des Verdauungstraktes, die sich von chronischer Gastritis, Diabetes mellitus, dem Zustand nach Magenresektion, gastrointestinalem Ulcus oder Krankheiten des Verdauungstraktes ableiten.

16. Verwendung einer Verbindung nach einem der Ansprüche 1 bis 11, bei der Herstellung eines Arzneimittels zur Verwendung bei der Behandlung von Rückfluss-Ösophagitis, irritablem Darmsyndrom oder falschem Darmverschluss.

## Revendications

1. Dérivé de diaminométhylidène représenté par la formule (1) 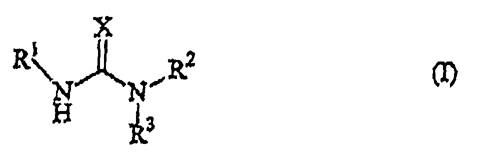 dans laquelle
R¹ est un atome d'hydrogène, un groups alkyle en C₁-C₆, un groupe cycloalkyle en C₃-C₆, un groupe (cycloalkyl en C₃-C₆)-alkyle en C₁-C₄, un groupe aryle ou un groupe aryl(alkyle en C₁-C₄), dans lequel le fragment aryle du groupe aryle ou du groupe aryl(alkyle en C₁-C₄) peut être substitué, le cas échéant, par un atome d'halogène, un groupe alkyle en C₁-C₆, un groups halogénoalkyle en C₁-C₆, un groupe alkoxy en C₁-C₆, un groupe (alkoxy en C₁-C₆)-carbonyle, un groupe phényle ou un groupe amino ;
X est O, S, CHNO₂, C(OOR⁴)₂, C(OOR⁴)CN ou C(CN)₂, dans lesquels R⁴ est un groupe alkyle en C₁-C₆, un groupe cycloalkyle en C₃-C₆, un groupe aryle ou un groups aryl(alkyle en C₁-C₄); et
R² est un groupe de formule générale (II) ou (III) suivante 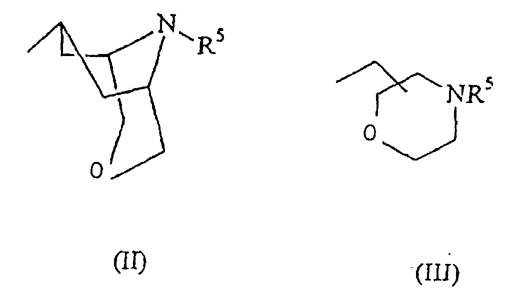 ou
X est CHNO₂, C(COOR⁴)₂, C(OOR⁴)CN ou C(CN)₂, dans lesquels R⁴ est tel que défini ci-dessus ; et
R² est un groupe de formule générale (IV) suivante 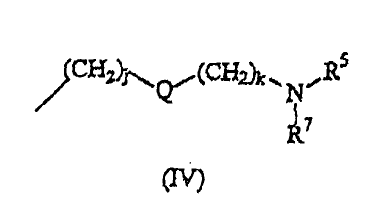 (dans laquelle j vaut 0-3, k vaut 0-3, et la somme de j et k vaut 1-6, et Q est O) ;
R³ est un atome d'hydrogène, un groups alkyle en C₁-C₆ ou un groupe alkoxy(en C₁-C₆)alkyle(en C₂-C₆) ;
R⁵ est un groupe aryl(alkyle en C₁-C₄), un groups hétéroaryl(alkyle en C₁-C₄), un groupe aryloxy(alkyle en C₂-C₆) ou un groupe pyrrolidinylcarbonyl(alkyle en C₁-C₄), dans lesquels le fragment aryle desdits groupes peut être substitué, le cas échéant, par un atome d'halogène, un groupe alkyle en C₁-C₆, un groupe halogéno(alkyle en C₁-C₆), un groupe alkoxy en C₁-C₆, un groupe (alkoxy en C₁-C₆)-carbonyle, un groupe phényle ou un groupe amino ; et
R⁷ est un atome d'hydrogène, un groupe (alkoxy en C₁-C₆)-alkyle (en C₂-C₄) ;
ou un groupe alkyle en C₁-C₄ ; ou un sel pharmacologiquement acceptable de celui-ci.

2. Composé selon la revendication 1, dans lequel R¹ est un groupe méthyle, isobutyle, cyclohexyle, phényle, p-méthoxyphényle, benzyle ou p-fluorobenzyle.

3. Composé selon la revendication 1 ou 2, dans lequel R³ est un atome d'hydrogène.

4. Composé selon l'une quelconque des revendications précédentes, dans lequel R⁵ est un groupe p-fluorobenzyle ou un groupe 3-(p-fluorophénoxy)propyle.

5. Composé selon la revendication 1, dans lequel
R¹ est un groupe méthyle, isobutyle, cyclohexyle, phényle ou benzyle,
X est C(COOC₂H₅)CN ou C(CN)₂,
R² est p-fluorobenzyl-2-morpholinylméthyle, et
R³ est un atome d'hydrogène.

6. Composé selon la revendication 1, dans lequel
R¹ est un groups p-méthoxyphényle,
X est un groupe C(COOC₂H₅)CN,
R² est p-fluorobenzyl-2-morpholinylméthyle et
R³ est un atome d'hydrogène.

7. Composé selon la revendication 1, dans lequel
R¹ est un groups p-fluorobenzyle,
X est un groups C(CN)₂,
R² est p-fluorobenzyl-2-morpholinylméthyle et
R³ est un atome d'hydrogène.

8. Composé selon la revendication 1, dans lequel
R¹ est un groupe méthyle,
X est O ou un groupe C(COOC₂H₅)CN, CNHO₂ ou C(CN)₂,
R² est un groupe de formule (II) dans laquelle R⁵ est un groupe p-fluorobenzyle et
R³ est un atome d'hydrogène.

9. Composé selon la revendication 1, dans lequel
R¹ est un groupe cyclohoxyle,
X est un groups C(COOC₂H₅)CN,
R² est un groupe 7-(p-fluorophényl)-6-aza-3-oxaheptyle et
R³ est un atome d'hydrogène.

10. Composé selon la revendication 1, dans lequel
R¹ est un groupe méthyle,
X est C(CN)₂,
R² est un composé de formule (II) dans laquelle R⁵ est un groupe 3-(p-fluorophénoxy)propyle et
R³ est un atome d'hydrogène.

11. Composé selon la revendication 1, dans lequel
R¹ est un groupe méthyle ou phényle,
X est O,
R² est un groupe de formule (III),
R⁵ est un groups p-fluorobenzyle et
R³ est un atome d'hydrogène.

12. Composition gastro-intestinale procinétique comprenant, comme ingrédient actif, un composé selon l'une quelconque des revendications précédentes, le cas échéant mélangé avec un additif pharmaceutiquement acceptable.

13. Composé selon l'une quelconque des revendications 1 à 11, à utiliser dans le traitement de troubles du tube digestif provenant d'une gastrite chronique, du diabète sucré, d'une post-gastrectomie ou d'un ulcère gastro-duodénal ou de maladies du tube digestif.

14. Composé selon l'une quelconque des revendications 1 à 11, à utiliser dans le traitement de l'oesophagite peptique, du syndrome de l'intestin irritable ou du pseudo-iléus.

15. Utilisation d'un composé salon l'une quelconque des revendications 1 à 11 pour la fabrication d'un médicament à utiliser dans le traitement de troubles du tube digestif provenant d'une gastrite chronique, du diabète sucré, d'une post-gastrectomie ou d'un ulcère gastro-duodénal ou de maladies du tube digestif.

16. Utilisation d'un composé selon l'une quelconque des revendications 1 à 11 pour la fabrication d'un médicament à utiliser dans le traitement de l'oesophagite peptique, du syndrome de l'intestin irritable ou du pseudo-iléus.
